(19) Europäisches Patentamt
European Patent Office
Office européen des brevets

(11) EP 4 806 925 A1

# (12) EUROPEAN PATENT APPLICATION

published in accordance with Art. 153(4) EPC

(43) Date of publication:
**16.09.2026 Bulletin 2026/38**

(21) Application number: **24887989.2**

(22) Date of filing: **06.11.2024**

(51) International Patent Classification (IPC):
***C08B 37/00*** *(2006.01)* ***C08B 37/10*** *(2006.01)*
***C08L 5/00*** *(2006.01)* ***A61K 31/737*** *(2006.01)*
***A61P 35/04*** *(2006.01)*

(52) Cooperative Patent Classification (CPC):
**A61K 31/727; A61K 31/737; A61P 35/00; A61P 35/04; C08B 37/00; C08B 37/0075; C08L 5/00**

(86) International application number:
**PCT/CN2024/130179**

(87) International publication number:
**WO 2025/098378 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.11.2023 CN 202311472694**

(71) Applicant: **Shenzhen Hepalink Pharmaceutical Group Co., Ltd.**
**Shenzhen, Guangdong 518057 (CN)**

(72) Inventors:
- **REN, Lige**
  **Shenzhen, Guangdong 518057 (CN)**
- **WANG, Jingwen**
  **Shenzhen, Guangdong 518057 (CN)**
- **JIN, Xuewen**
  **Shenzhen, Guangdong 518057 (CN)**
- **ZHANG, Yunhao**
  **Shenzhen, Guangdong 518057 (CN)**
- **HUANG, Baoming**
  **Shenzhen, Guangdong 518057 (CN)**
- **FENG, Heini**
  **Shenzhen, Guangdong 518057 (CN)**
- **LI, Li**
  **Shenzhen, Guangdong 518057 (CN)**

(74) Representative: **Gille Hrabal Partnerschaftsgesellschaft mbB**
**Patentanwälte**
**Brucknerstraße 20**
**40593 Düsseldorf (DE)**

## (54) CHEMICALLY MODIFIED GLYCOSAMINOGLYCAN, PREPARATION METHOD THEREFOR, AND USE THEREOF

(57) The present disclosure provides a chemically modified glycosaminoglycan, which has good anti-tumor activity, good safety, and excellent druggability and has good physical/chemical stability by introducing a galacturonic acid structure through a reaction and controlling an appropriate ring opening degree on the basis of retaining a part of the six-membered ring structure of the glycosaminoglycan. The chemically modified glycosaminoglycan of the present disclosure has good anti-tumor activity, particularly anti-tumor metastasis and anti-tumor growth activity, has good stability, stable metabolism, good bioavailability, improved pharmacokinetic properties, small toxic and side effects, and good safety, and can be prepared into a suitable preparation, thereby improving the drug safety, and improving the patient compliance and the convenience of administration.

1800
1600
1400
1200
1000
800
600
400
200
0
Tumor volume (mm³)
1
4
8
11
15
18
22
25
Days of experiment (days)
Control group
Sample from Comparative Example 1 20 mg/kg
Sample from Comparative Example 1 40 mg/kg
Sample from Comparative Example 1 80 mg/kg
Sample D from Example 4 20 mg/kg
Sample D from Example 4 40 mg/kg
Sample D from Example 4 80 mg/kg
FIG. 3

EP 4 806 925 A1

## Description

The present application claims priority to:

a prior application with the application No. 202311472694.4 entitled "CHEMICALLY MODIFIED GLYCOSAMINOGLYCAN, PREPARATION METHOD THEREFOR, AND USE THEREOF" and filed with China National Intellectual Property Administration on Nov. 07, 2023,
which is incorporated herein by reference in its entirety.

## TECHNICAL FIELD

The present disclosure relates to a chemically modified glycosaminoglycan (in particular, a carboxylated glycosaminoglycan derivative), a manufacturing method therefor, and use thereof for inhibiting tumor growth and/or metastasis.

## BACKGROUND

With rapid economic development, aggravation of environmental pollution and changes in people's lifestyle, malignant tumors have become diseases that seriously endanger life and health of people in China and restrict economic development in the society. Among all causes of death, malignant tumors have become the leading cause of death in China in recent years.

In the development of most malignant tumors, distant metastasis is the leading cause of death in cancer patients. The steps of tumor metastasis mainly include detachment of the tumor from the primary site, vascular infiltration, transportation within the blood vessel, adhesion to the vascular endothelium, extravasation from the blood vessel, and finally formation of distant metastasis. It has been reported in the literature that vascular endothelial cells, fibroblasts, tumor-associated immune cells, extracellular matrix, inflammation-associated cytokines and other components in the tumor microenvironment play an important role in the invasion, metastasis and progression of tumors, but the specific mechanisms have not been fully understood.

Heparanase is an endo-$\beta$-glucuronase that is capable of cleaving heparan sulfate (HS) in heparan sulfate proteoglycans, such as syndecan-1, thereby releasing growth factors bound to heparan sulfate.

Extensive research work over the past two decades has shown that heparanase expression is upregulated in a variety of human cancers, sarcomas, and hematological malignant tumors. Studies also suggest that elevated heparanase levels may be associated with late-stage progression and metastasis of many tumor types. For example, high levels of heparanase are associated with shorter post-operative survival time of patients. The direct role of heparanase in tumor metastasis has been demonstrated in the laboratories of Vlodavsky and Sanderson.

Heparin is a linear, polydisperse, sulfated polysaccharide of the glycosaminoglycan family that has anticoagulant and antithrombotic activities. The saccharide chains of heparin is consisted of alternating uronic acids and D-glucosamine, with the major repeating units being 2-O-sulfated L-iduronic acid (IdoA2S) and $\alpha(1\rightarrow4)$ N-, 6-O-disulfated D-glucosamine (GlcN6S) and the minor components being non-sulfated L-iduronic acid and D-glucuronic acid, and N-acetyl D-glucosamine and N-, 3-O-, 6-O-trisulfated D-glucosamine (Casu B., 2005. "Structure and active domains of heparin." In: Chemistry and biology of heparin and heparan sulfate. Amsterdam: Elsevier. 1-28; Casu B. and Lindahl U., 2001, "Structure and biological interactions of heparin and heparan sulfate." Adv Carbohydr Chem Biochem, 57: 159-206). Heparin can effectively inhibit heparanase, but due to the strong anticoagulant activity of heparin, it is impossible to use heparin at high doses as a therapeutic agent for tumors. The risk of bleeding is a necessary indicator for assessing the safety of anticoagulant drugs and particularly heparin analogues.

A class of non-anticoagulant heparins that can be used as heparanase inhibitors has been disclosed in the prior art, most of which comprise structurally modified non-sulfated uronic acid residues. Such structures can be obtained by cleaving carbon-carbon bonds at positions 2-3 on glycosaminoglycan residues (glycol splitting) to open the glycosidic ring. However, the non-anticoagulant heparins in the prior art have relatively low efficacy or safety and need to be further improved.

In conclusion, there is an urgent technical problem to be solved to provide a medicament with high safety and high inhibitory activity against tumor metastasis.

## SUMMARY

The present disclosure provides a chemically modified glycosaminoglycan, which has good anti-tumor activity, particularly has good anti-tumor growth activity, anti-tumor metastasis activity and safety, and particularly has excellent anti-tumor metastasis activity and safety.

### Chemically Modified Glycosaminoglycan

The present disclosure provides a chemically modified glycosaminoglycan comprising a structural unit of formula (I), a structural unit of formula (II), a structural unit of formula (III), and a structural unit of formula (IV):

formula (I), formula (II),

formula (III), formula (IV);

wherein:

- $R^{1m}$, $R^{1n}$, and $R^{1q}$, at each occurrence, are each independently selected from H, $-SO_3^-$, and $-(C{=}O)CH_3$, and preferably, $R^{1m}$ and $R^{1n}$ , at each occurrence, are $-SO_3^-$ or $-(C{=}O)CH_3$;
- $R^{2m}$, $R^{2n}$, $R^{2q}$, and $R^{2x}$, at each occurrence, are each independently selected from H and $-SO_3^-$;
- $R^{3n}$, at each occurrence, is each independently selected from H and $-SO_3^-$;
- $R^{4m}$ and $R^{4n}$, at each occurrence, are each independently selected from H and $-SO_3^-$.

According to an embodiment of the present disclosure, the chemically modified glycosaminoglycan has a weight-average molecular weight of 10,000 Da to 15,000 Da, preferably 10,500 Da to 14,000 Da, such as 10,500 Da, 11,000 Da, 11,500 Da, 12,000 Da, 12,500 Da, 13,000 Da, 13,500 Da, and 14,000 Da.

According to an embodiment of the present disclosure, the chemically modified glycosaminoglycan has a content of galacturonic acid of 0.10%-2.00%, preferably 0.3%-1.90%, 0.5%-1.80%, 0.70%-1.70%, and 1.10%-1.40%, e.g., 0.7%, 0.80%, 0.90%, 1.00%, 1.10%, 1.20%, 1.30%, 1.40%, 1.60%, and 1.70%.

According to an embodiment of the present disclosure, the chemically modified glycosaminoglycan has a ring-opening degree of uronic acid of 45%-75%, preferably 48%-72% and 50%-70%, more preferably 53%-68%, and further preferably 53%-63%, such as 53%, 54%, 55%, 56%, 57%, 58%, 59%, 60%, 61%, 62%, 63%, 64%, 65%, 67%, 68%, 69%, and 70%.

According to an embodiment of the present disclosure, the chemically modified glycosaminoglycan has a sulfo-carboxyl ratio ($SO_3^-/COO^-$) of 0.7-1.5, preferably 0.8-1.3, such as 0.8, 0.9, 1.0, 1.1, 1.2, and 1.3.

According to an embodiment of the present disclosure, the chemically modified glycosaminoglycan has a carboxyl increment of 1.20-2.00, preferably 1.60-1.90, and further preferably 1.65-1.75, such as 1.60, 1.65, 1.70, 1.75, 1.80, 1.85, and 1.90.

According to an embodiment of the present disclosure, the chemically modified glycosaminoglycan has a polydispersity index of 1.00-1.24, preferably 1.05-1.24 or 1.05-1.22, and further preferably 1.10-1.20, such as 1.10, 1.11, 1.12, 1.13, 1.14, 1.15, 1.16, 1.17, 1.18, and 1.19.

According to an embodiment of the present disclosure, the chemically modified glycosaminoglycan has a epoxy degree of uronic acid of less than 10%, such as less than 8% or less than 6%, preferably 0%-5%, 0.05%-4%, 0.1%-3%, 0.5%-2.5%, or 0.7%-2.3%, e.g., 0.5%, 0.6%, 0.7%, 0.8%, 0.9%, 1.0%, 1.1%, 1.2%, 1.8%, 1.9%, 2.0%, 2.1%, 2.2%, 2.3%, 2.4%, and 2.5%.

According to an embodiment of the present disclosure, the molecular weight distribution of the chemically modified glycosaminoglycan is as follows:

| | |
|---|---|
| greater than 19,000 Da | 0-20% (such as 0.5%, 1%, 3%, 6%, 9%, 10%, 15%, 16%, 17%, and 18%) |
| 6,000-19,000 Da 89%) | 73%-90% (such as 74%, 75%, 76%, 77%, 78%, 79%, 80%, 83%, 85%, 86%, 87%, 88%, and |
| less than 6,000 Da | 2%-15% (such as 3%, 4%, 5%, 6%, 8%, 10%, 11%, 12%, and 13%). |

According to an embodiment of the present disclosure, the molecular weight distribution of the chemically

modified glycosaminoglycan is as follows:

| Molecular weight range (Da) | Proportion (% by weight) |
|---|---|
| Greater than 19,000 | 0~18 |
| 6,000-19,000 | 73~90 |
| Less than 6,000 | 3-15 |

Preferably, the molecular weight distribution of the chemically modified glycosaminoglycan is as follows:

| Molecular weight range (Da) | Proportion (% by weight) |
|---|---|
| Greater than 19,000 | 1-18 (such as 5-10) |
| 6,000-19,000 | 78~89 |
| Less than 6,000 | 3-13 (such as 5-10) |

According to an embodiment of the present disclosure, the structural unit of formula (I), the structural unit of formula (II), the structural unit of formula (III), and the structural unit of formula (IV) are statistically distributed along the polysaccharide chain.

According to an embodiment of the present disclosure, the chemically modified glycosaminoglycan optionally further comprises a structural unit of formula (V):

formula (V)

wherein:

$R^{2t}$ and $R^{3t}$, at each occurrence, are each independently selected from H and $-SO_3^-$.

According to an embodiment of the present disclosure, the chemically modified glycosaminoglycan optionally further comprises a structural unit of formula (VI):

formula (VI)

wherein:

$R^{2p}$, at each occurrence, is each independently selected from H and $-SO_3^-$.

According to an embodiment of the present disclosure, the chemically modified glycosaminoglycan optionally further comprises a structural unit of formula (VII):

formula (VII)

wherein:
$R^{2r}$ and $R^{3r}$, at each occurrence, are each independently selected from H and $-SO_3^-$.

According to an embodiment of the present disclosure, the chemically modified glycosaminoglycan optionally further comprises a structural unit of formula (VIII):

formula (VIII)

wherein:

$R^{1s}$, at each occurrence, is each independently selected from H, $-SO_3^-$, and $-(C=O)CH_3$, preferably $-SO_3^-$ or $-(C=O)CH_3$;
$R^{2s}$ and $R^{4s}$, at each occurrence, are each independently selected from H and $-SO_3^-$;
$R^{3s}$, at each occurrence, is $-SO_3^-$.

Optionally, one or more polysaccharide chains of the chemically modified glycosaminoglycan may each comprise m structural unit(s) of formula (I), wherein m is selected from any integer from 1 to 25, inclusive; preferably selected from any integer from 11 to 16, inclusive.

Optionally, one or more polysaccharide chains of the chemically modified glycosaminoglycan may each comprise n structural unit(s) of formula (II), wherein n is selected from any integer from 1 to 10, inclusive; preferably selected from any integer from 1 to 8, inclusive.

Optionally, one or more polysaccharide chains of the chemically modified glycosaminoglycan may each comprise q structural unit(s) of formula (III), wherein q is selected from any integer from 1 to 15, inclusive; preferably selected from any integer from 1 to 10, inclusive.

Optionally, one or more polysaccharide chains of the chemically modified glycosaminoglycan may each comprise x structural unit(s) of formula (IV), wherein x is selected from any integer from 0 to 10, inclusive; preferably selected from any integer from 0 to 8, inclusive; and more preferably selected from any integer from 1 to 8, inclusive.

Optionally, one or more polysaccharide chains of the chemically modified glycosaminoglycan may each comprise t structural unit(s) of formula (V), wherein t is selected from any integer from 0 to 6, inclusive; preferably selected from any integer from 0 to 4, inclusive; and more preferably selected from any integer from 0 to 3, inclusive. Optionally, one or more polysaccharide chains of the chemically modified glycosaminoglycan may each comprise p structural unit(s) of formula (VI), wherein p is selected from any integer from 0 to 8, inclusive; preferably selected from any integer from 0 to 4, inclusive; and more preferably selected from any integer from 0 to 2, inclusive. Optionally, one or more polysaccharide chains of the chemically modified glycosaminoglycan may each comprise r structural unit(s) of formula (VII), wherein r is selected from any integer from 0 to 8, inclusive; preferably selected from any integer from 0 to 4, inclusive.

Optionally, one or more polysaccharide chains of the chemically modified glycosaminoglycan may each comprise s structural unit(s) of formula (VIII), wherein s is selected from any integer from 1 to 10, inclusive; preferably selected from any integer from 1 to 8, inclusive.

In each polysaccharide chain of the chemically modified glycosaminoglycan of the present disclosure, the subject structural units are arranged in a random order.

According to an embodiment of the present disclosure, in the chemically modified glycosaminoglycan, the number of the structural unit of formula (I) is greater than the sum of the numbers of the structural units of formula (V) and formula (VI).

According to an embodiment of the present disclosure, in the chemically modified glycosaminoglycan, the ratio of the number of the structural unit of formula (I) to the sum of the numbers of the structural units of formula (I), formula (V), and formula (VI) is greater than 0.8.

According to an embodiment of the present disclosure, in the chemically modified glycosaminoglycan, the ratio of the number of the structural unit of formula (I) to the sum of the numbers of the structural units of formula (I) and formula (II) is 0.4-0.8.

According to an embodiment of the present disclosure, in the chemically modified glycosaminoglycan, the ratio of the number of the structural unit of formula (IV) to the sum of the numbers of the structural units of formula (I), formula (II), formula (III), and formula (IV) is less than 0.2.

The present disclosure encompasses any combination of the embodiments.

**Method for Preparing Chemically Modified Glycosaminoglycan**

The present disclosure provides a method for preparing the chemically modified glycosaminoglycan, which comprises the following steps:

a) epoxidizing C2, C3 of the uronic acid residue in the starting material glycosaminoglycan, preferably in the presence of a base (such as sodium hydroxide or potassium hydroxide);
b) hydrolysing the epoxidation product obtained in step a) to perform ring opening, preferably under a neutral condition;
c) under a condition effective to convert adjacent diols (vicinal diols) and optionally adjacent $OH/NH_2$ into dialdehydes, oxidizing 10%-100% (preferably 25%-100%) 2-O- and optionally 2-N-, 3-O-unsulfated residues of the glycosaminoglycan;
wherein the oxidation is performed in the presence of an oxidant selected from one of periodic acid and periodate (such as sodium periodate, or potassium periodate, etc.); and
d) under a condition effective to convert the dialdehydes into carboxyl groups and without nitrogen protection, further oxidizing the product obtained in step c),
wherein the further oxidation is preferably performed by a chlorite (such as sodium chlorite, magnesium chlorite $Mg(ClO_2)_2$, or barium chlorite $Ba(ClO_2)_2$, etc.);
e) separating and purifying the product of step d) by an anion exchange purification system to obtain the chemically modified glycosaminoglycan.

According to an embodiment of the present disclosure, the method for preparing the chemically modified glycosaminoglycan takes glycosaminoglycan as a starting material, performs C2, C3 epoxidation and ring opening under controlled conditions, effectively converts adjacent dihydroxy groups and optionally adjacent $OH/NH_2$ into aldehyde via a controlled glycol splitting reaction, and then forms a corresponding carboxyl group of the corresponding aldehyde group under suitable oxidation conditions.

According to an embodiment of the present disclosure, the glycosaminoglycan is natural heparin or synthetic heparin (optionally chemically or enzymatically modified) from any animal and organ sources, preferably selected from optionally 2-O- and/or 2-N-desulfated heparin, unfractionated heparin (UFH), low molecular weight heparin (LMWH, with a molecular weight of 3,500-8,000 Da), and heparan sulfate (HS); the glycosaminoglycan has a sulfo-carboxyl ratio of 0.8-2.8 (preferably 0.9-2.5); more preferably, the glycosaminoglycan is selected from optionally 2-O- and/or 2-N-desulfated unfractionated heparin and low molecular weight heparin.

According to an embodiment of the present disclosure, in step a), the glycosaminoglycan has a weight-average molecular weight of 10,000 Da to 30,000 Da, preferably 15,000 Da to 25,000 Da, such as 15,000 Da to 20,000 Da, 15,000 Da to 19,000 Da, or 17,000 Da to 19,000 Da.

According to an embodiment of the present disclosure, the heparin chain may naturally comprise about 5% to 35% 2-O-unsulfated uronic acid residues, 0% to 50% N-acetylated glucosamine residues, and about 0% to 6% N-unsubstituted (neither N-sulfated nor N-acetylated) glucosamine residues. The different sulfation degree depends on the heparin source (animal species and organ source) and the extraction procedure.

Each 2-O- or 2-N-unsulfated residue (without the 3-O-sulfate substituent) of the glycosaminoglycan is easy to subject to ring-opening (splitting) reaction, and under oxidation conditions, the vicinal diol and $OH/NH_2$ can undergo conversion to aldehyde.

According to an embodiment of the present disclosure, in step a), by adjusting the reaction conditions, e.g., controlling the reaction time and/or the amount of base, more uronic acids in the glycosaminoglycan are subjected to an epoxidation reaction. For example, 50%-70% of the uronic acid structural units are subjected to an epoxidation reaction.

According to an embodiment of the present disclosure, in step a), the ratio of the molar amount of the base to the mass of the starting material glycosaminoglycan is 3.0 mmol/g to 15.0 mmol/g and 5.0 mmol/g to 12.0 mmol/g, preferably 6.0-7.0 mmol/g, such as 6.0 mmol/g, 6.2 mmol/g, 6.3 mmol/g, 6.5 mmol/g, 7.0 mmol/g, 8.0 mmol/g, 9.0 mmol/g, 10.0 mmol/g, and 11.0 mmol/g.

According to an embodiment of the present disclosure, in step a), the reaction temperature is 55-75 °C, preferably 60-70 °C, such as 55-60 °C, 60-65 °C, 65-70 °C, and 70-75 °C.

According to an embodiment of the present disclosure, in step a), the reaction time is 80-800 min, preferably 120-700 min, and further preferably 240-600 min, such as 240 min, 360 min, 480 min, and 600 min.

According to an embodiment of the present disclosure, in step a), after the reaction is completed, the reaction solution is cooled to room temperature and adjusted to neutral (pH 6.5-7.5).

According to an embodiment of the present disclosure, in step b) of the method, the hydrolysis reaction is performed under neutral conditions to yield a galacturonic acid structure.

According to an embodiment of the present disclosure, in step b), a relatively large amount of the epoxidation

product obtained in step a) is subjected to a hydrolysis reaction by adjusting reaction conditions, such as controlling the reaction time and the reaction temperature. In some embodiments, the epoxy degree of uronic acid in the hydrolysis product obtained in step b) is controlled to be less than 10%, e.g., less than 8%, or 0-6%, preferably 0.05%-5%.

According to an embodiment of the present disclosure, in step b), the reaction temperature is 60-85 °C, preferably 65-75 °C, such as 60-65 °C, 65-70 °C, 70-75 °C, 70-75 °C, 75-80 °C, and 80-85 °C.

According to an embodiment of the present disclosure, in step b), the reaction time is 20-150 h or 36-96 h, preferably 48-72 h, such as 36 h, 48 h, 60 h, 72 h, and 96 h.

According to an embodiment of the present disclosure, in step b), after the reaction is completed, the reaction solution is cooled to room temperature.

According to an embodiment of the present disclosure, in step c), the ratio of the molar amount of the oxidant to the mass of the starting material glycosaminoglycan is 0.1 mmol/g to 10.0 mmol/g, 0.5 mmol/g to 8.0 mmol/g, or 1.0 mmol/g to 5.0 mmol/g, preferably 1.5 mmol/g to 4.0 mmol/g, such as 2.0 mmol/g, 2.6 mmol/g, 2.8 mmol/g, 3.0 mmol/g, 3.2 mmol/g, 3.4 mmol/g, 3.6 mmol/g, and 3.7 mmol/g.

According to an embodiment of the present disclosure, in step c), the reaction temperature is 0 °C to 20 °C.

According to an embodiment of the present disclosure, in step c), the reaction time is 5-50 h or 10-30 h, preferably 12-24 h, such as 12 h, 14 h, 16 h, 17 h, 18 h, 20 h, and 24 h.

According to an embodiment of the present disclosure, step c) is performed in a dark condition.

According to an embodiment of the present disclosure, in step c), after the reaction is completed, an excess amount of a termination reagent is added to quench the reaction; optionally, the reaction solution is further subjected to dialysis (for desalting).

According to an embodiment of the present disclosure, in step c), the termination reagent is selected from one or more of ethylene glycol, ethanolamine, glycerol, glucose, mannose, glyceraldehyde, threose, and xylose; preferably, the termination reagent is selected from one or more of ethylene glycol, ethanolamine, and glucose.

According to an embodiment of the present disclosure, in step c), the amount of the termination reagent is, for example, 10-20 mL.

In step c) of the method, a part of vicinal diols in the uronic acid is not cleaved by controlling the glycol splitting reaction, such as controlling the amount of an oxidant or the reaction time, so that the galacturonic acid structural fragment can be introduced into the final product. The acidic glycosaminoglycan containing the galacturonic acid structural fragment has related properties such as good activity and safety.

According to an embodiment of the present disclosure, in step d), the ratio of the molar amount of the chlorite (e.g., sodium chlorite, magnesium chlorite $Mg(ClO_2)_2$, barium chlorite $Ba(ClO_2)_2$, etc.) to the mass of the starting material glycosaminoglycan is 3.0-20.0 mmol/g or 6.0-13.0 mmol/g, preferably 7.0-12.0 mmol/g, such as 8.0 mmol/g, 9.0 mmol/g, 10.0 mmol/g, and 11.0 mmol/g.

According to an embodiment of the present disclosure, in step d), the reaction temperature is 0 °C to 15 °C. According to an embodiment of the present disclosure, in step d), the reaction pH is 3.0-5.0, preferably 3.5-4.5, such as 3.5, 4.0, and 4.5.

According to an embodiment of the present disclosure, in step d), the reaction time is 5-100 h or 12-36 h, preferably 18-30 h, such as 16 h, 18 h, 20 h, 22 h, 24 h, 26 h, 28 h, and 30 h.

According to an embodiment of the present disclosure, after the reaction in step d) is completed, the pH of the reaction solution is adjusted to be neutral, ethanol is added, and the mixture is left to stand to give a precipitate. According to an embodiment of the present disclosure, the precipitate obtained in step d) has a polydispersity index of 1.10-1.40, e.g., 1.20-1.30, such as 1.21, 1.22, 1.23, 1.24, 1.25, 1.26, 1.27, 1.28, and 1.29.

According to an embodiment of the present disclosure, the precipitate obtained in step d) has a content of galacturonic acid of 0.10%-2.00%, 0.3%-1.90%, 0.5%-1.80%, 0.50%-1.70%, 0.7%-1.4%, or 1.10%-1.40%, such as 0.7%, 0.80%, 0.90%, 1.00%, 1.10%, 1.20%, 1.30%, 1.40%, 1.60%, and 1.70%.

According to an embodiment of the present disclosure, in step e), after separation and purification, the obtained chemically modified glycosaminoglycan has a polydispersity index of 1.00-1.24, e.g., 1.10-1.20, such as 1.10, 1.11, 1.12, 1.13, 1.14, 1.15, 1.16, 1.17, 1.18, and 1.19.

According to an embodiment of the present disclosure, in step e), after separation and purification, the obtained chemically modified glycosaminoglycan has a weight-average molecular weight of 10,000 Da to 15,000 Da, preferably 10,500 Da to 14,000 Da, such as 10,500 Da, 11,000 Da, 11,500 Da, 12,000 Da, 12,500 Da, 13,000 Da, 13,500 Da, and 14,000 Da.

According to an embodiment of the present disclosure, in step e), after separation and purification, the molecular weight distribution of the obtained chemically modified glycosaminoglycan is as follows:

| | |
|---|---|
| greater than 19,000 Da | 0-20% |
| 6,000-19,000 Da | 73%-90% |

(continued)

| | |
|---|---|
| less than 6,000 Da | 2%-15%. |

In some embodiments, in step e), the product (precipitate) obtained in step d) is dissolved in an acidic aqueous solution, and the mixture is loaded and separated and purified by an anion exchange purification system to obtain the chemically modified glycosaminoglycan.

In some embodiments, in step e), the acidic aqueous solution is selected from one or more of an aqueous acetic acid solution, an aqueous phosphoric acid solution, an aqueous citric acid solution, and an aqueous sodium dihydrogen phosphate solution; the concentration of the acidic aqueous solution is 0.05%-10% (w/w%), preferably 0.2%-2% (w/w%).

In some embodiments, in step e), the anion exchange purification system is one of a weak anion exchange purification system and a strong anion exchange purification system; preferably, the anion exchange purification system is a weak anion exchange purification system; preferably, the chromatographic column packing of the anion exchange purification system is selected from one of DEAE Sepharose Fast Flow, ANX Sepharose 4 FF, and DEAE Seplife FF.

In some embodiments, in step e), the chromatographic conditions for the anion exchange purification system include: the mobile phase A is an acidic solution; the acid in the acidic solution is, for example, selected from one or more of acetic acid, phosphoric acid, citric acid, and sodium dihydrogen phosphate; for example, the acid in the acidic solution is acetic acid. In some embodiments, the acidic solution is an acidic aqueous solution. In some embodiments, the concentration of the acidic solution is 0.05%-10% (w/w%), e.g., 0.2%-2% (w/w%) or 0.1%-0.5% (w/w%). In some embodiments, the mobile phase A is a 0.3% aqueous acetic acid solution.

In some embodiments, in step e), the chromatographic conditions for the anion exchange purification system include: the mobile phase B is an acidic solution containing a salt, wherein the salt is, for example, selected from one or more of sodium chloride, sodium acetate, disodium hydrogen phosphate, and sodium perchlorate. In some embodiments, the concentration of the salt in the mobile phase B is 0.5-3 mol/L. In some embodiments, the acid in the acidic solution is selected from one or more of acetic acid, phosphoric acid, citric acid, and sodium dihydrogen phosphate; for example, the acid in the acidic solution is acetic acid. In some embodiments, the acidic solution is an acidic aqueous solution. In some embodiments, the concentration of the acidic solution is 0.05%-10% (w/w%), e.g., 0.2%-2% (w/w%) or 0.1%-0.5% (w/w%). In some embodiments, the mobile phase B is a 0.1-0.5% (e.g., 0.3%) aqueous acetic acid solution containing 0.5-3 mol/L (e.g., 2 mol/L) sodium chloride.

Preferably, the detector used in the anion exchange purification system is selected from one of an ultraviolet detector and a conductivity detector.

In some embodiments, in step e), the instrument of the anion exchange purification system includes: a preparative high-performance liquid chromatograph (e.g., SCG-030) and an ultraviolet detector (e.g., a DAD 600EX ultraviolet detector).

In some embodiments, in step e), the chromatographic conditions for the anion exchange purification system include: the packing of the chromatographic column is DEAE Sepharose Fast Flow with a particle size of, e.g., 90 μm, and a dimension of, e.g., 50 mm × 100 mm;

preferably, the mobile phase A is a 0.1-0.5% (e.g., 0.3%) aqueous acetic acid solution, and the mobile phase B is a 0.1-0.5% (e.g., 0.3%) aqueous acetic acid solution containing 0.5-3.0 mol/L (e.g., 2.0 mol/L) sodium chloride;
preferably, the average linear speed of the mobile phase is 0.1-5.0 cm/min; further preferably, the average linear speed of the mobile phase is 0.5-2.0 cm/min or 0.8-1.2 cm/min (e.g., 1.0 cm/min);
preferably, sample loading is performed at room temperature;
preferably, the run time is 300 minutes;
preferably, the gradient elution conditions are shown in Table 1:

Table 1

| Elution process No. | Time (min) | Mobile phase A (%) | Mobile phase B (%) |
|---|---|---|---|
| 1 | 0~110 | 75~80 | 20~25 |
| 2 | 110~210 | 55~60 | 40~45 |
| 3 | 210~300 | 0 | 100 |

In some embodiments, in step e), in elution process 1, the mobile phase B (%) is 20%, 21%, 22%, 23%, 24%, and 25%.

In some embodiments, in step e), in elution process 2, the mobile phase B (%) is 40%, 41%, 42%, 43%, 44%, and 45%.

In some embodiments, in step e), the eluate in elution process 2 (110-210 min) is collected, adjusted to pH

6.0-7.0, desalted, concentrated, and precipitated with ethanol to give the chemically modified glycosaminoglycan.

In some embodiments, in step e), the eluate in elution process 2 (110-210 min) is collected, adjusted to pH 6.0-7.0, desalted, concentrated, precipitated with ethanol, dissolved with water, and lyophilized to give the chemically modified glycosaminoglycan.

In some embodiments, the chemically modified glycosaminoglycan exhibits a carboxyl increment of 1.3-2.0, wherein the carboxyl increment is calculated as the ratio of the sulfation degree of the starting material to the sulfation degree of the glycosaminoglycan. More specifically, the sulfation degree of the starting material is determined after reduction with $NaBH_4$ of a sample of the glycosaminoglycan intermediate obtained after subjecting the starting material to the first oxidation step (step c). The specific procedure for calculating the carboxyl increment can be found in CN105744940A.

The present disclosure further provides a chemically modified glycosaminoglycan manufactured by the manufacturing method described above.

In the present disclosure, "compound of the present disclosure", "chemically modified glycosaminoglycan", and "chemically modified glycosaminoglycan of the present disclosure" are used interchangeably and all refer to a chemically modified glycosaminoglycan as described above.

The term "sulfation degree", as used herein, refers to the sulfo-carboxyl ratio ($SO_3^-/COO^-$ molar ratio), which is determined by conductimetric titration according to Casu B. and Gennaro U., 1975, Carbohydr Res 39, 168-176.

The term "carboxyl increment", as used herein, refers to the ratio of the sulfation degree of the starting material to the sulfation degree of the carboxylated derivative (the chemically modified glycosaminoglycan). More specifically, the sulfation degree of the starting material is the sulfation degree determined after reduction of the sample of the glycosaminoglycan intermediate obtained after the first oxidation step (step (c)). The sulfation degree of the chemically modified glycosaminoglycan is the sulfation degree determined in the second oxidation step and after purification (step (d) and step (e)). The specific procedure for calculating the carboxyl increment can be found in CN105744940A.The term "ring-opening degree of uronic acid", as used herein, refers to the number of ring-opening uronic acid residues/the number of total uronic acid residues.

The term "epoxy degree of uronic acid", as used herein, refers to the ratio of the uronic acid structural unit with an epoxy structure in formula (IV) to the total uronic acid residues; for their detection and calculation, reference was made to the nuclear magnetic resonance method in the document Guerrini, M., Guglieri, S., Naggi, A., Sasisekharan, R., & Torri, G. (2007). Low molecular weight heparins: Structural differentiation by bidimentional nuclear magnetic resonance spectroscopy. Seminars in Thrombosis and Hemostasis, 33, 478-487.

The term "content of galacturonic acid", as used herein, refers to the relative content of galacturonic acid, i.e., the ratio of galacturonic acid structural units to N-acetylated glucosamine structural units. Since the reactions of the present disclosure do not involve a change in the acetyl group of the N-acetylated glucosamine and thus the number of acetylated glucosamine remains substantially unchanged during the reactions of the present disclosure, the content of galacturonic acid is characterized using N-acetylated glucosamine as a standard (i.e., an internal standard). The calculation formula for the content of galacturonic acid in the present disclosure is as follows: content of galacturonic acid = (number of galacturonic acid residues/number of N-acetylated glucosamine residues) $\times$ 100%. The term "polydispersity index", as used herein, is a parameter that characterizes the dispersibility of a substance and is the ratio of the weight-average molecular weight to the number-average molecular weight (i.e., PDI = Mw/Mn). The smaller the PDI value, the more uniform the molecular weight in the system, and the better the system quality. The PDI of an absolutely monodisperse system is equal to 1, and it is generally considered that PDI less than 1.2 can be regarded as narrow dispersion.

## Pharmaceutical Composition and Treatment Method

The present disclosure provides a pharmaceutical composition comprising a prophylactically or therapeutically effective amount of the chemically modified glycosaminoglycan of the present disclosure and one or more pharmaceutically acceptable carriers. The pharmaceutical composition is preferably in a form of a solid, semi-solid, liquid, or gas preparation.

In some embodiments, the pharmaceutical composition may further comprise one or more additional therapeutic agents.

The present disclosure provides use of the chemically modified glycosaminoglycan of the present disclosure or the pharmaceutical composition of the present disclosure for the manufacture of a medicament for the inhibition of tumor growth and/or metastasis.

The present disclosure provides use of the chemically modified glycosaminoglycan or the pharmaceutical composition described above for the manufacture of a medicament for the treatment and/or prevention of a tumor/cancer.

The present disclosure provides the chemically modified glycosaminoglycan of the present disclosure or the pharmaceutical composition of the present disclosure for use in inhibiting tumor growth and/or metastasis.

The present disclosure provides the chemically modified glycosaminoglycan described above or the pharma-

ceutical composition described above for use in treating and/or preventing a tumor/cancer.

The present disclosure provides a method for inhibiting tumor growth and/or metastasis, comprising administering to a subject in need thereof an effective amount of the glycosaminoglycan of the present disclosure or the pharmaceutical composition of the present disclosure.

In some embodiments, the tumor is a solid tumor, a hematological tumor, or a soft tissue tumor, preferably a solid tumor, e.g., breast cancer, pancreatic cancer, bladder cancer, prostate cancer, colon cancer, gastric cancer, or lung cancer.

The present disclosure provides a method for treating and/or preventing a tumor/cancer, comprising administering to a subject in need thereof an effective amount of the glycosaminoglycan of the present disclosure or the pharmaceutical composition of the present disclosure.

In some embodiments, the tumor is a solid tumor, a hematological tumor, or a soft tissue tumor, preferably a solid tumor, e.g., breast cancer, pancreatic cancer, bladder cancer, prostate cancer, colon cancer, gastric cancer, or lung cancer.

The "pharmaceutically acceptable carrier" in the present disclosure refers to a diluent, an adjuvant, an excipient, or a vehicle administered together with a therapeutic agent, which is suitable, within the scope of sound medical judgment, for contact with the tissues of humans and/or other animals without undue toxicity, irritation, allergic response, or other problems or complications relative to a reasonable benefit/risk ratio.

The pharmaceutically acceptable carriers that can be used in the pharmaceutical composition of the present disclosure include, but are not limited to, a sterile liquid, e.g., water and oil, including those of petroleum, animal, vegetable, or synthetic source, e.g., peanut oil, soybean oil, mineral oil, sesame oil, and the like. Water is an exemplary carrier when the pharmaceutical composition is administered intravenously. Normal saline, glucose, and an aqueous glycerol solution may also be used as a liquid carrier, particularly for an injectable liquid. Suitable pharmaceutical excipients include starch, glucose, lactose, sucrose, gelatin, maltose, chalk, silica gel, sodium stearate, glyceryl monostearate, talc, sodium chloride, dried skim milk, glycerol, propylene glycol, water, ethanol, and the like. The composition may further optionally contain a small amount of wetting agent, emulsifier, or pH buffer, as needed. Oral preparation can include standard carriers such as pharmaceutical grades of mannitol, lactose, starch, magnesium stearate, sodium saccharine, cellulose, magnesium carbonate, etc. Examples of suitable pharmaceutically acceptable carriers are described in Remington's Pharmaceutical Sciences (1990).

The pharmaceutical composition of the present disclosure may act systemically and/or topically. For this purpose, they may be administered by any suitable route, for example, by injection (e.g., intravenous, intraarterial, subcutaneous, intraperitoneal, and intramuscular injection, including drip infusion) or transdermally; or orally, buccally (oral holding), nasally, transmucosally, topically, in an ophthalmic preparation, or by inhalation.

For these routes of administration, the pharmaceutical composition of the present disclosure may be administered in suitable dosage forms.

The dosage form includes, but is not limited to, tablets, capsules, lozenges, hard candies, powders, sprays, creams, ointments, suppositories, gels, pastes, lotions, ointments, aqueous suspensions, injectable solutions, elixirs, and syrups.

The term "effective amount" as used herein refers to an amount of a derivative that, after administration, will relieve to some extent one or more of the symptoms of the condition being treated.

The dosing regimen may be adjusted to provide the optimum desired response. For example, a single dose may be administered, several separate doses may also be administered over time, or the dose may be proportionally decreased or increased as needed by the treatment situation. It is noted that the dose may vary with the type and severity of the condition to be alleviated, and may include single or multiple doses. It is further understood that for any particular subject, the specific dosage regimen will be adjusted over time according to the subject need and the professional judgment of the person administering the composition or supervising the administration of the composition.

The amount of the chemically modified glycosaminoglycan of the present disclosure administered will depend on the subject being treated, the severity of the disorder or condition being treated, the rate of administration, the configuration of the glycosaminoglycan, and the judgment of the prescribing physician. Generally, an effective dose is about 0.0001 mg to about 50 mg/kg/day, e.g., about 0.01 mg/kg/day to about 10 mg/kg/day (single or separate administration). For a person weighing 70 kg, this would total about 0.007 mg/day to about 3,500 mg/day, for example, about 0.7 mg/day to about 700 mg/day. In some cases, dosage levels not higher than the lower limit of the above range may be sufficient, while in other cases, a larger dosage may still be employed without causing any harmful side effects, provided that the larger dosage is first divided into several smaller dosages for administration throughout the day.

The content or amount of the chemically modified glycosaminoglycan of the present disclosure in the pharmaceutical composition may be about 0.01 mg to about 1,000 mg, suitably 0.1 mg to 500 mg, preferably 0.5 mg to 300 mg, more preferably 1 mg to 150 mg, and particularly preferably 1 to 50 mg, for example, 1.5 mg, 2 mg, 4 mg, 10 mg, 25 mg, etc.

As used herein, unless otherwise indicated, the term "treat", "treating", or "treatment" refers to reversing, alleviating, or inhibiting the progression of a disorder or condition of a disease or one or more symptoms of a disorder or

condition of a disease, or preventing a disorder or condition of a disease or one or more symptoms of a disorder or condition of a disease.

As used herein, "subject" includes humans or non-human animals. Exemplary human subjects include human subjects with a disease (e.g., a disease described herein), which are referred to as patients, or normal subjects. In the present disclosure, "non-human animals" include all vertebrates, such as non-mammals (e.g., birds, amphibians, reptiles) and mammals, such as non-human primates, livestock and/or domesticated animals (e.g., sheep, dogs, cats, cows, pigs, etc.).

Beneficial effects

The present disclosure provides a chemically modified glycosaminoglycan, which has good anti-tumor activity, good safety, and excellent druggability and has good physical/chemical stability by introducing a galacturonic acid structure through a reaction and controlling an appropriate ring opening degree on the basis of retaining a part of the six-membered ring structure of the glycosaminoglycan. The chemically modified glycosaminoglycan of the present disclosure has good anti-tumor activity, particularly anti-tumor metastasis and anti-tumor growth activity, has good stability, stable metabolism, good bioavailability, improved pharmacokinetic properties, small toxic and side effects, and good safety, and can be prepared into a suitable preparation, thereby improving the drug safety, and improving the patient compliance and the convenience of administration.

## BRIEF DESCRIPTION OF THE DRAWINGS

FIG. 1 shows the molecular weight distributions of intermediate 4j and sample J from Example 10.

FIG. 2 shows the nuclear magnetic resonance HSQC spectra of sample J from Example 10 and the sample from Comparative Example 1. FIGs. 2a and 2b are nuclear magnetic resonance HSQC spectra of sample J and the sample from Comparative Example 1, respectively. FIG. 2c is an overlay of the nuclear magnetic resonance HSQC spectra of sample J and the sample from Comparative Example 1, where the gray spectrum is the nuclear magnetic resonance HSQC spectrum of sample J from Example 10, and the black spectrum is the nuclear magnetic resonance HSQC spectrum of the sample from Comparative Example 1.

FIG. 3 shows the tumor growth curves of the test samples in the experiment of Test Example 3 of the present application on the subcutaneous xenograft tumor of human breast cancer xenograft cells MDA-MB-231.

FIG. 4 shows the tumor sizes of the test samples in the experiment of Test Example 3 of the present application on the subcutaneous xenograft tumor of human breast cancer xenograft cells MDA-MB-231.

FIG. 5 shows the tumor growth curves of the test samples in the experiment of Test Example 4 of the present application on the subcutaneous xenograft tumor of human pancreatic cancer xenograft cells Mia PaCa-2.

FIG. 6 shows the tumor sizes of the test samples in the experiment of Test Example 4 of the present application on the subcutaneous xenograft tumor of human pancreatic cancer xenograft cells Mia PaCa-2.

FIG. 7 shows the tumor growth curves of the test samples in the experiment of Test Example 5 of the present application on the subcutaneous xenograft tumor of murine myeloma cells MPC-11.

FIG. 8 shows the time-tumor growth curves of the test samples in the experiment of Test Example 6 of the present application on the subcutaneous xenograft tumor of human mesothelioma xenograft cells MSTO-211H.

FIG. 9 shows the time-concentration curves of each drug in rat plasma after subcutaneous injection of the compound of the present disclosure and the sample from Comparative Example 1 in the experiment of Test Example 7 of the present application.

FIG. 10 shows the survival rate of mice after subcutaneous injection of the compound of the present disclosure and the sample from Comparative Example 1 in the experiment of Test Example 9 of the present application.

## DETAILED DESCRIPTION

The technical solutions of the present disclosure are further described in detail below with reference to specific examples. It should be understood that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be manufactured by using known methods.

Examples and experimental examples are listed below to further describe the present disclosure in detail.

However, they are not intended to limit the scope of the present disclosure, and changes may be made without departing from the scope of the present disclosure.

The abbreviations or acronyms used herein have the following meanings: $NaIO_4$ for sodium periodate, and $NaClO_2$ for sodium chlorite.

Summary: C2 and C3 epoxidation and ring opening are performed under controlled conditions using a glycosaminoglycan (such as unfractionated heparin or low molecular weight heparin) as a starting material to obtain a glycosaminoglycan containing a galacturonic acid structure. The adjacent dihydroxy groups and optionally adjacent $OH/NH_2$ are then converted to aldehyde via a controlled glycol splitting reaction followed by oxidation of the corresponding aldehyde group to form the corresponding carboxyl group.

Glycosaminoglycan: glycosaminoglycan is a heteropolysaccharide that is an unbranched, long-chain polymer composed of repeating complex units containing hexuronic acid and hexosamine components.

Unfractionated heparin (UFH), also known as normal heparin, is a sulfated glycosaminoglycan compound with a molecular weight of generally 3,000-30,000 Daltons (Da). It is an inhomogeneous polysaccharide formed by linking disaccharide units formed by uronic acid (L-iduronic acid, IdoA and D-glucuronic acid, GlcA) and hexosamine (α-D-glucosamine, GlcN) and their derivatives (acetylated and sulfated).

Low molecular weight heparin (LMWH): it is manufactured from unfractionated heparin through different degradation processes. It has similar monosaccharide composition, substitution pattern, and oligosaccharide sequence structural characteristics to heparin, and has a lower molecular weight than that of normal heparin. Chromatographic conditions for the weak anion exchange purification system used in examples of the present disclosure are as follows: Instrument: preparative liquid phase chromatograph (SCG-030) and ultraviolet detector (DAD 600EX ultraviolet detector).

Specification of chromatographic column: packing: DEAE Sepharose Fast Flow, particle size: 90 μm, dimension (D/L): 50 mm × 100 mm.

Elution conditions: the mobile phase A was a 0.3% aqueous acetic acid solution, and the mobile phase B was a 0.3% aqueous acetic acid solution containing 2 mol/L sodium chloride; the average linear speed of the mobile phase was 1.0 cm/min (the average linear speed of a mobile phase is the average speed of the mobile phase moving along the axial direction of the chromatographic column, which is the average length of the mobile phase flowing through the chromatographic column per unit time, in cm/min); the sample was loaded at room temperature; the run time was 300 min; the gradient elution conditions are shown in Table 1:

Table 1

| Elution process No. | Time (min) | Proportion of mobile phase A (%) | Proportion of mobile phase B (%) |
|---|---|---|---|
| 1 | 0~110 | 75~80 | 20~25 |
| 2 | 110~210 | 55~60 | 40~45 |
| 3 | 210~300 | 0 | 100 |
| Note: the sum of the proportions of mobile phase A and mobile phase B is 100%. | | | |

The molecular weight distribution, polydispersity index, etc. were determined by gel permeation chromatography (GPC) using the following instruments and conditions:

The instruments included an EMPOWER workstation, an ultra-high performance liquid chromatograph (ACQUITY UPLC), and a detector (differential refractometer detector). The chromatographic column used was ACQUITY APC AQ, with a particle size of 2.5 μm and a dimension (D/L) of 4.6 mm × 150 mm. The injection volume was 10 μL; the mobile phase was a 0.1 M ammonium acetate solution containing 0.02% sodium azide, the flow rate was 0.5 mL/min, and isocratic elution was performed; the collection time was 10 min.

The content of galacturonic acid in the present disclosure was determined by HSQC. The calculation formula for the content of galacturonic acid was as follows: content of galacturonic acid = (galacturonic acid characteristic signal peak intensity/C-H correlation signal peak intensity at the acetyl group at the C2 position of N-acetylated glucosamine) × 100%, and the intensity of each C-H correlation signal peak was determined using HSQC. HSQC: heteronuclear single quantum correlation (HSQC), belonging to one of NMR (nuclear magnetic resonance) C-H COSY spectra. The information given by HSQC is the directly linked hydrocarbon relationship C-H. In the present application, the HSQC method was used to measure the intensity of C-H correlation signal peaks.

In the HSQC spectrum of the present application, the C-H correlation signal peak at the acetyl group at the C2 position of N-acetylglucosamine had a chemical shift value of δ (2.07+0.10, 104.8+1.0); the characteristic signal peak of galacturonic acid was the C-H correlation signal peak at the C5 position of galacturonic acid, which had a chemical shift value of δ (4.71±0.10, 74.4±1.0).

In HSQC, for the assignment of the chemical shift of the nuclear magnetic resonance HSQC peak of heparin sodium and low-molecular-weight heparin sodium, reference was made to the document "Qualification of HSQC methods for quantitative composition of heparin and low molecular weight heparins" (Mauri, L., et al., Journal of Pharmaceutical and Biomedical Analysis, 2017, 136: 92-105).

The numbering scheme for the pyranose ring C in glucopyranose is shown in the following chemical structural formula. In the present disclosure, the numbering for C in the uronic acid and glucosamine was performed with reference to the numbering scheme for C in the pyranose ring in the formula below.

6
CH2OH
5
O
1
HO
OH
OH
4
3
2
OH
Numbering scheme for C in pyranose ring

Example 1

(1) weighed 5 g (Mw = 18306 Da) of unfractionated heparin and dissolved in 31.2 g of purified water, and the mixture was preheated to 60 °C. An NaOH solution (1 mol/L, 31.3 mL) was added. The reaction solution was heated to 55-60 °C and reacted for 600 min. After the reaction, the solution was cooled to room temperature and adjusted to neutrality (pH 6.5-7.5) with an HCl solution (10%-19%, w/w).

(2) The solution obtained in step (1) was heated to 60-65 °C, hydrolyzed for 96 h under neutral conditions, and cooled to room temperature after the reaction.

(3) At 0-20 °C, a sodium periodate solution (0.2 mol/L, 75 mL) was added to the reaction solution obtained in step (2). The mixture was stirred and reacted for 17 h in the dark. After the reaction, 10.0 mL of ethylene glycol was added, and stirring was continued for 1 h to terminate the reaction. Dialysis was performed for 24 h (desalting) to give a dialysate.

(4) At 0-15 °C, a sodium chlorite solution (0.67 mol/L, 75 mL) was added to the dialysate obtained in step (3), and glacial acetic acid was added to adjust the pH to about 3.5. The mixture was stirred and reacted for 18 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4a, which had a polydispersity index of 1.249 and a galacturonic acid content of 1.26%.

(5) The precipitate obtained in step (4) was dissolved in a 0.3% aqueous acetic acid solution, and the sample was quantitatively loaded and purified by the weak anion exchange purification system. The separation conditions are described in the analytical method of the weak anion exchange purification system described above, wherein the proportion of the mobile phase B in elution process 1 was 20%, and the proportion of the mobile phase B in elution process 2 was 40%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample A. The yield was 63.3%.

Example 2

(1) 5 g (Mw = 17,000 Da) of unfractionated heparin was weighed out and dissolved in 31.3 g of purified water, and the mixture was preheated to 60 °C. An NaOH solution (1 mol/L, 31.3 mL) was added. The reaction solution was heated to 60-65 °C and reacted for 360 min. After the reaction, the solution was cooled to room temperature and adjusted to neutrality (pH 6.5-7.5) with an HCl solution (10%-19%, w/w).

(2) The solution obtained in step (1) was heated to 65-70 °C, hydrolyzed for 72 h under neutral conditions, and cooled to room temperature after the reaction.

(3) At 0-20 °C, a sodium periodate solution (0.2 mol/L, 85 mL) was added to the reaction solution obtained in step (2). The mixture was stirred and reacted for 12 h in the dark. After the reaction, 20.0 mL of ethylene glycol was added, and stirring was continued for 1 h to terminate the reaction. Dialysis and desalting were performed to give a dialysate.

(4) At 0-15 °C, a sodium chlorite solution (0.67 mol/L, 85 mL) was added to the dialysate obtained in step (3), and glacial acetic acid was added to adjust the pH to about 4.0. The mixture was stirred and reacted for 20 h, and the pH was adjusted to neutrality by adding an NaOH solution (30% w/v). Ethanol was added for precipitation, and the mixture was left to stand for 24 h. The supernatant was discarded to obtain a solid precipitate. The resulting precipitate was designated as intermediate 4b, which had a polydispersity index of 1.263 and a galacturonic acid content of 1.55%.
(5) The precipitate obtained in step (4) was dissolved in a 0.3% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 25%, and the proportion of the mobile phase B in elution process 2 was 45%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample B. The yield was 78.5%.

Example 3

(1) 5 g (Mw = 17,000 Da) of unfractionated heparin was weighed out and dissolved in 31.3 g of purified water, and the mixture was preheated to 60 °C. An NaOH solution (1 mol/L, 31.3 mL) was added. The reaction solution was heated to 55-60 °C and reacted for 480 min. After the reaction, the solution was cooled to room temperature and adjusted to neutrality (pH 6.5-7.5) with an HCl solution (10%-19%, w/w).
(2) The solution obtained in step (1) was heated to 65-70 °C, hydrolyzed for 72 h under neutral conditions, and cooled to room temperature after the reaction.
(3) At 0-20 °C, a sodium periodate solution (0.2 mol/L, 80 mL) was added to the reaction solution obtained in step (2). The mixture was stirred and reacted for 14 h in the dark. After the reaction, 15.0 mL of ethylene glycol was added, and stirring was continued for 1 h to terminate the reaction. Dialysis was performed for 24 h (desalting) to give a dialysate.
(4) At 0-15 °C, a sodium chlorite solution (0.67 mol/L, 80 mL) was added to the dialysate obtained in step (3), and glacial acetic acid was added to adjust the pH to about 4.0. The mixture was stirred and reacted for 22 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4c, which had a polydispersity index of 1.265 and a galacturonic acid content of 1.43%.
(5) The precipitate obtained in step (4) was dissolved in a 0.5% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 23%, and the proportion of the mobile phase B in elution process 2 was 43%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample C. The yield was 71.7%.

Example 4

(1) 8 g (Mw = 18150 Da) of unfractionated heparin was weighed out and dissolved in 50.1 g of purified water, and the mixture was preheated to 60 °C and dissolved in an NaOH solution (1 mol/L, 50.3 mL) was added. The reaction solution was heated to 60-65 °C and reacted for 240 min. After the reaction, the solution was cooled to room temperature and adjusted to neutrality (pH 6.5-7.5) with an HCl solution (10%-19%, w/w).
(2) The solution obtained in step (1) was heated to 70-75 °C, hydrolyzed for 48 h under neutral conditions, and cooled to room temperature after the reaction.
(3) At 0-20 °C, a sodium periodate solution (0.2 mol/L, 120 mL) was added to the reaction solution obtained in step (2). The mixture was stirred and reacted for 16 h in the dark. After the reaction, 16 mL of ethylene glycol was added, and stirring was continued for 1 h to terminate the reaction. Dialysis was performed for 24 h (desalting) to give a dialysate.
(4) At 0-15 °C, a sodium chlorite solution (0.67 mol/L, 120 mL) was added to the dialysate obtained in step (3), and glacial acetic acid was added to adjust the pH to about 4.0. The mixture was stirred and reacted for 24 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4d, which had a polydispersity index of 1.256 and a galacturonic acid content of 1.12%.
(5) The precipitate obtained in step (4) was dissolved in a 0.3% aqueous acetic acid solution, and the sample was

quantitatively loaded and separated and purified by the weak anion exchange purification system. The separation conditions are described in the analytical method of the weak anion exchange purification system described above, wherein the proportion of the mobile phase B in elution process 1 was 21%, and the proportion of the mobile phase B in elution process 2 was 41%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample D. The yield was 66.7%.

Example 5

(1) 5 g (Mw = 17675 Da) of unfractionated heparin was weighed out and dissolved in 31.3 g of purified water, and the mixture was preheated to 60 °C. An NaOH solution (1 mol/L, 50.5 mL) was added. The reaction solution was heated to 60-65 °C and reacted for 80 min. After the reaction, the solution was cooled to room temperature and adjusted to neutrality (pH 6.5-7.5) with an HCl solution (10%-19%, w/w).
(2) The solution obtained in step (1) was heated to 75-80 °C, hydrolyzed for 48 h under neutral conditions, and cooled to room temperature after the reaction.
(3) At 0-20 °C, a potassium periodate solution (0.2 mol/L, 90 mL) was added to the reaction solution obtained in step (2). The mixture was stirred and reacted for 20 h in the dark. After the reaction, 10.0 mL of ethylene glycol was added, and stirring was continued for 1 h to terminate the reaction. Dialysis and desalting were performed for 24 h to give a dialysate.
(4) At 0-15 °C, a sodium chlorite solution (0.67 mol/L, 90 mL) was added to the dialysate obtained in step (3), and glacial acetic acid was added to adjust the pH to about 4.5. The mixture was stirred and reacted for 16 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4e, which had a polydispersity index of 1.246 and a galacturonic acid content of 0.69%.
(5) The precipitate obtained in step (4) was dissolved in a 0.3% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 23%, and the proportion of the mobile phase B in elution process 2 was 43%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample E. The yield was 70.5%.

Example 6

(1) 10 g (Mw = 17806 Da) of unfractionated heparin was weighed out and dissolved in 62.5 g of purified water, and the mixture was preheated to 60 °C. An NaOH solution (1 mol/L, 62.5 mL) was added. The reaction solution was heated to 60-65 °C and reacted for 120 min. After the reaction, the solution was cooled to room temperature and adjusted to neutrality (pH 6.5-7.5) with an HCl solution (10%-19%, w/w).
(2) The solution obtained in step (1) was heated to 80-85 °C, hydrolyzed for 36 h under neutral conditions, and cooled to room temperature after the reaction.
(3) At 0-20 °C, a sodium periodate solution (0.2 mol/L, 185 mL) was added to the reaction solution obtained in step (2). The mixture was stirred and reacted for 16 h in the dark. After the reaction, 20.0 mL of ethylene glycol was added, and stirring was continued for 1 h to terminate the reaction. Dialysis was performed for 24 h (desalting) to give a dialysate.
(4) At 0-15 °C, a sodium chlorite solution (0.67 mol/L, 185 mL) was added to the dialysate obtained in step (3), and glacial acetic acid was added to adjust the pH to about 5.0. The mixture was stirred and reacted for 30 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4f, which had a polydispersity index of 1.252 and a galacturonic acid content of 0.75%.
(5) The precipitate obtained in step (4) was dissolved in a 0.3% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 21%, and the proportion of the mobile phase B in elution process 2 was 41%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and

lyophilized to give sample F. The yield was 64.8%.

Example 7

(1) 5 g (Mw = 18306 Da) of unfractionated heparin was weighed out and dissolved in 31.3 g of purified water, and the mixture was preheated to 60 °C and added to an NaOH solution (1 mol/L, 31.3 mL). The reaction solution was heated to 65-70 °C and reacted for 240 min. After the reaction, the solution was cooled to room temperature and adjusted to neutrality (pH 6.5-7.5) with an HCl solution (10%-19%, w/w).
(2) The solution obtained in step (1) was heated to 70-75 °C, hydrolyzed for 72 h under neutral conditions, and cooled to room temperature after the reaction.
(3) At 0-20 °C, a sodium periodate solution (0.2 mol/L, 65 mL) was added to the reaction solution obtained in step (2). The mixture was stirred and reacted for 16 h in the dark. After the reaction, 10.0 mL of ethylene glycol was added, and stirring was continued for 1 h to terminate the reaction. Dialysis was performed for 24 h (desalting) to give a dialysate.
(4) At 0-15 °C, a sodium chlorite solution (0.67 mol/L, 65 mL) was added to the dialysate obtained in step (3), and glacial acetic acid was added to adjust the pH to about 4.0. The mixture was stirred and reacted for 28 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4g, which had a polydispersity index of 1.244 and a galacturonic acid content of 1.36%.
(5) The precipitate obtained in step (4) was dissolved in a 0.3% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 23%, and the proportion of the mobile phase B in elution process 2 was 43%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample G. The yield was 73.2%.

Example 8

(1) 5 g (Mw = 18306 Da) of unfractionated heparin was weighed out and dissolved in 31.3 g of purified water, and the mixture was preheated to 60 °C. An NaOH solution (1 mol/L, 31.3 mL) was added. The reaction solution was heated to 65-70 °C and reacted for 240 min. After the reaction, the solution was cooled to room temperature and adjusted to neutrality (pH 6.5-7.5) with an HCl solution (10%-19%, w/w).
(2) The solution obtained in step (1) was heated to 65-70 °C, hydrolyzed for 48 h under neutral conditions, and cooled to room temperature after the reaction.
(3) At 0-20 °C, a sodium periodate solution (0.2 mol/L, 65 mL) was added to the reaction solution obtained in step (2). The mixture was stirred and reacted for 16 h in the dark. After the reaction, 10.0 mL of ethylene glycol was added, and stirring was continued for 1 h to terminate the reaction. Dialysis was performed for 24 h (desalting) to give a dialysate.
(4) At 0-15 °C, a sodium chlorite solution (0.67 mol/L, 65 mL) was added to the dialysate obtained in step (3), and glacial acetic acid was added to adjust the pH to about 4.0. The mixture was stirred and reacted for 24 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4h, which had a polydispersity index of 1.261 and a galacturonic acid content of 1.32%.
(5) The precipitate obtained in step (4) was dissolved in a 0.3% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 21%, and the proportion of the mobile phase B in elution process 2 was 41%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample H. The yield was 68.6%.

Example 9

(1) 5 g (Mw = 18306 Da) of unfractionated heparin was weighed out and dissolved in 62.5 g of purified water, and the mixture was preheated to 60 °C and added to an NaOH solution (1 mol/L, 60 mL). The reaction solution was heated to 70-75 °C and reacted for 240 min. After the reaction, the solution was cooled to room temperature and adjusted to neutrality (pH 6.5-7.5) with an HCl solution (10%-19%, w/w).

(2) The solution obtained in step (1) was heated to 70-75 °C, hydrolyzed for 72 h under neutral conditions, and cooled to room temperature after the reaction.

(3) At 0-20 °C, a sodium periodate solution (0.2 mol/L, 50 mL) was added to the reaction solution obtained in step (2). The mixture was stirred and reacted for 24 h in the dark. After the reaction, 10.0 mL of ethylene glycol was added, and stirring was continued for 1 h to terminate the reaction. Dialysis was performed for 24 h (desalting) to give a dialysate.

(4) At 0-15 °C, a sodium chlorite solution (0.67 mol/L, 50 mL) was added to the dialysate obtained in step (3), and glacial acetic acid was added to adjust the pH to about 3.0. The mixture was stirred and reacted for 12 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4i, which had a polydispersity index of 1.265 and a galacturonic acid content of 1.65%.

(5) The precipitate obtained in step (4) was dissolved in a 0.3% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 23%, and the proportion of the mobile phase B in elution process 2 was 43%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample I. The yield was 75.1%.

Example 10

(1) 5 g (Mw = 18306 Da) of unfractionated heparin was weighed out and dissolved in 31.3 g of purified water, and the mixture was preheated to 60 °C and added to an NaOH solution (1 mol/L, 31.3 mL). The reaction solution was heated to 65-70 °C and reacted for 240 min. After the reaction, the solution was cooled to room temperature and adjusted to neutrality (pH 6.5-7.5) with an HCl solution (10%-19%, w/w).

(2) The solution obtained in step (1) was heated to 70-75 °C, hydrolyzed for 72 h under neutral conditions, and cooled to room temperature after the reaction.

(3) At 0-20 °C, a sodium periodate solution (0.2 mol/L, 50 mL) was added to the reaction solution obtained in step (2). The mixture was stirred and reacted for 18 h in the dark. After the reaction, 10.0 mL of ethylene glycol was added, and stirring was continued for 1 h to terminate the reaction. Dialysis was performed for 24 h (desalting) to give a dialysate.

(4) At 0-15 °C, a sodium chlorite solution (0.67 mol/L, 50 mL) was added to the dialysate obtained in step (3), and glacial acetic acid was added to adjust the pH to about 4.0. The mixture was stirred and reacted for 26 h, and the pH was adjusted to neutrality with an NaOH solution (30% w/v). Ethanol was added for precipitation, and the mixture was left to stand for 24 h. The supernatant was discarded to obtain a precipitate. The resulting precipitate was designated as intermediate 4j, which had a polydispersity index of 1.256 and a galacturonic acid content of 1.67%.

(5) The precipitate obtained in step (4) was dissolved in a 0.3% aqueous acetic acid solution, and the sample was quantitatively loaded and separated and purified by the weak anion exchange purification system. The separation conditions are described in the analytical method of the weak anion exchange purification system described above, wherein the proportion of the mobile phase B in elution process 1 was 21%, and the proportion of the mobile phase B in elution process 2 was 41%. The eluate of 110-210 min was collected, adjusted to pH 6.0-7.0 with an NaOH solution (30%, w/w) or an HCl solution (10%-19%), desalted, concentrated, precipitated with ethanol, dissolved with water, and lyophilized to give sample J. The yield was 70.3%.

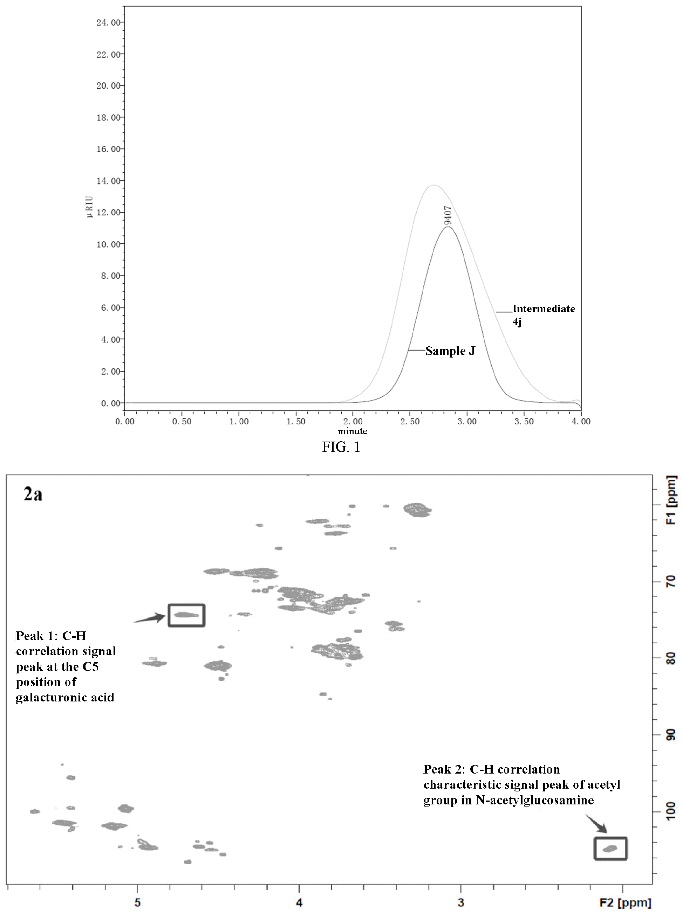

The molecular weight distribution of intermediate 4j and sample J was determined by gel permeation chromatography (GPC) and is shown in FIG. 1. The polydispersity index of the refined sample J was 1.116, and the polydispersity index of intermediate 4j before refining was 1.256; after the refining step of step (5), the molecular weight distribution of sample J was significantly narrowed, demonstrating higher product uniformity; the polydispersity index of the refined product was less than 1.20, indicating superior product quality.

Comparative Example 1

The sample of Comparative Example 1 was prepared according to the method described in Example 10 of WO2019149179A1. The specific method was as follows:

1) 5 g (Mw = 18306 Da) of unfractionated heparin was weighed out and dissolved in 62.5 g of a 1 mol/L NaOH solution. The reaction solution was heated to 60 °C and reacted for 30 min. After the reaction, the solution was cooled at room temperature and adjusted to neutrality with a 1:1 HCl solution (commercially available 36%-38% concentrated hydrochloric acid and purified water were formulated in a volume ratio of 1:1).

2) The solution of step 1) was heated to 70 °C and hydrolyzed for 24 h under neutral conditions. After the reaction, the mixture was cooled to room temperature.

3) At a low temperature, 100 g of a 0.2 mol/L $NaIO_4$ solution was added to the reaction solution obtained in step 2). The mixture was stirred for 16 h with the temperature controlled at 4 °C in the dark. After the reaction, 10.0 mL of ethylene glycol was added, and stirring was continued for 1 h to terminate the reaction. The reaction solution was dialyzed.

4) At a low temperature, 100 g of a 6.03% (m/v) $NaClO_2$ solution was added to the reaction solution obtained in step 3), and the pH was adjusted to about 4.0 with glacial acetic acid. The mixture was stirred and reacted at room temperature for 24 h, and adjusted to neutrality with a 30% (w/v) NaOH solution. The reaction solution was dialyzed for 24 h, and the dialysate was concentrated by rotary evaporation and lyophilized to obtain a sample of Comparative Example 1 with Mw = 11,445 Da, $SO_3^-/COO^-$ = 1.21, a carboxyl increment of 1.64, a ring-opening degree of uronic acid of 33.2%, and an epoxy degree of uronic acid of 17.6%. The polydispersity index was 1.249, and the galacturonic acid content was 0.05%.

Comparative Example 2

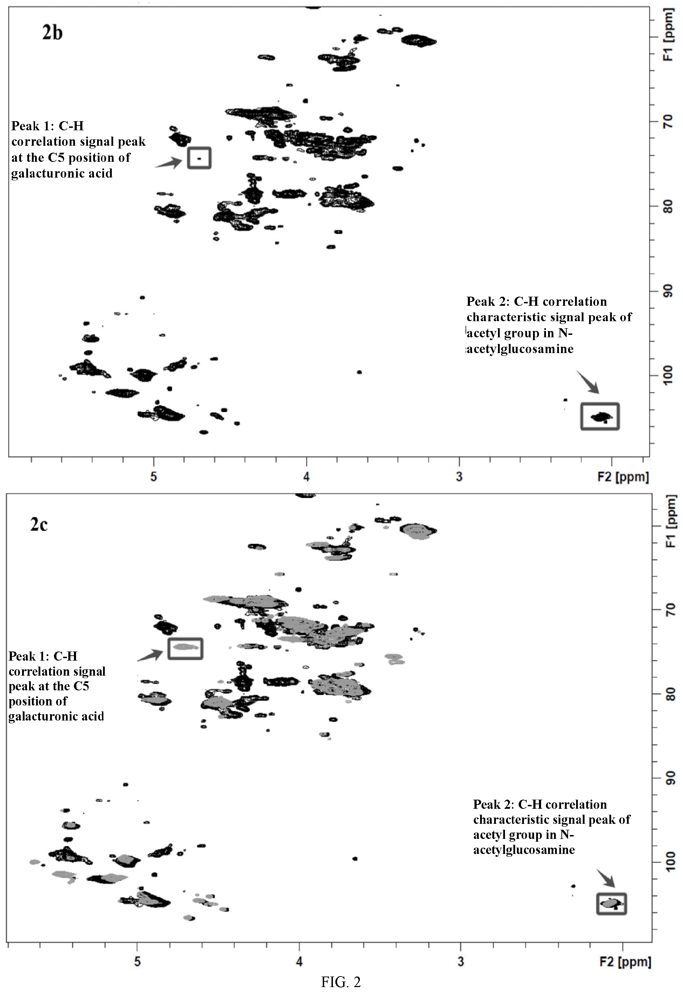

Sample J from Example 10 and the sample from Comparative Example 1 were subjected to nuclear magnetic resonance HSQC spectrum testing. The nuclear magnetic resonance HSQC spectra of sample J and the sample of Comparative Example 1 are shown in FIG. 2a and FIG. 2b, respectively. The obtained nuclear magnetic resonance HSQC spectra of the two samples were overlaid, which is shown in FIG. 2c. In FIG. 2c, the gray spectra are nuclear magnetic resonance HSQC spectra of sample J from Example 10, and the black spectra are nuclear magnetic resonance HSQC spectra of the sample from Comparative Example 1. The chemical shift value of the C-H correlation signal peak at the C5 position of galacturonic acid was $\delta$ (4.71$\pm$0.10, 74.4+1.0), and the chemical shift value of the C-H correlation signal peak of the acetyl group at the N-acetylated glucosamine was $\delta$ (2.07+0.10, 104.8$\pm$1.0). It can be clearly seen from the figure that sample J contained a large amount of galacturonic acid, while the sample from Comparative Example 1 contained almost no galacturonic acid (peak 1). The content of galacturonic acid was characterized by the ratio of A/B, where the integral of the C-H correlation HSQC spectrum (peak 1) at the C5 position of galacturonic acid was A, and the integral of the C-H correlation HSQC spectrum (peak 2) at the acetyl group of N-acetylated glucosamine was B. Sample J from Example 10 had a content of galacturonic acid of 1.68%, and the sample from Comparative Example 1 had a content of galacturonic acid of 0.05% (see Table 2).

The products obtained in Examples 1-10 and Comparative Example 1 of the present disclosure were tested. Their characterization data and molecular weight distribution are shown in Table 2. The polydispersity indexes of the intermediates and final products obtained in step (4) of Examples 1-10 of the present disclosure are shown in Table 3 below.

Table 2

| Sample No. | Weight-average molecular weight | >19,000 | 6,000-19,000 | <6,000 | $SO_3^-$ /$COO^-$ | Carboxyl increment | % | Content of galacturonic acid% | Polydispersity index | Epoxy degree of uronic acid% |
|---|---|---|---|---|---|---|---|---|---|---|
| A | 10558 | 0.9% | 86.5% | 12.6% | 1.0 | 1.76 | 63.5 | 1.24 | 1.112 | 0.7 |
| B | 13887 | 17.7% | 78.8% | 3.5% | 1.1 | 1.71 | 65.6 | 1.51 | 1.132 | 1.8 |
| C | 12587 | 10.3% | 85.9% | 3.8% | 1.0 | 1.73 | 64.2 | 1.4 | 1.119 | 1.2 |
| D | 11268 | 6.1% | 88.2% | 5.7% | 1.0 | 1.75 | 62.5 | 1.14 | 1.115 | 1.9 |
| E | 12165 | 9.8% | 85.4% | 4.8% | 0.8 | 1.88 | 68.2 | 0.72 | 1.121 | 2.1 |
| F | 10603 | 1.0% | 88.5% | 10.5% | 0.8 | 1.89 | 67.2 | 0.77 | 1.118 | 0.6 |
| G | 13008 | 15.2% | 78.3% | 6.5% | 1.2 | 1.72 | 57.1 | 1.35 | 1.126 | 0.7 |
| H | 11987 | 8.8% | 84.9% | 6.3% | 1.2 | 1.68 | 56.6 | 1.3 | 1.119 | 2.3 |
| I | 13325 | 15.6% | 79.3% | 5.1% | 1.2 | 1.68 | 53.9 | 1.64 | 1.127 | 0.5 |
| J | 12245 | 8.7% | 86.0% | 5.3% | 1.2 | 1.66 | 53.3 | 1.68 | 1.116 | 0.8 |
| Sample of Comparative Example 1 | 11445 | 22.90% | 63.30% | 13.80% | 1.21 | 1.64 | 33.2 | 0.05 | 1.249 | 17.6 |

Table 3

| No. | Step (4) intermediate | | Final product | |
|---|---|---|---|---|
| | Content of galacturonic acid% | Polydispersity index | Content of galacturonic acid% | Polydispersity index |
| Example 1 | 1.26 | 1.249 | 1.24 | 1.112 |
| Example 2 | 1.55 | 1.263 | 1.51 | 1.132 |
| Example 3 | 1.43 | 1.265 | 1.4 | 1.119 |
| Example 4 | 1.12 | 1.256 | 1.14 | 1.115 |
| Example 5 | 0.69 | 1.246 | 0.72 | 1.121 |
| Example 6 | 0.75 | 1.252 | 0.77 | 1.118 |
| Example 7 | 1.36 | 1.244 | 1.35 | 1.126 |
| Example 8 | 1.32 | 1.261 | 1.3 | 1.119 |
| Example 9 | 1.65 | 1.265 | 1.64 | 1.127 |
| Example 10 | 1.67 | 1.256 | 1.68 | 1.116 |

By comparing the compound of the present disclosure with the sample from Comparative Example 1, it was found that the products of the present disclosure had a high degree of molecular weight distribution concentration, the molecular weight being mainly distributed in the range of 6,000-19,000. The ring-opening degrees of uronic acid of the products of the present disclosure were all 50%-70%, which were much higher than 33.2% of the sample from Comparative Example 1. The polydispersity indexes of the products of the present disclosure were all lower than 1.20, indicating good polydispersity. The galacturonic acid content of the products of the present disclosure was significantly higher than that of the sample from Comparative Example 1, which was 14.4-33.6 times that of the sample from Comparative Example 1.

In conclusion, there were significant differences between the products of the present disclosure and the sample from Comparative Example 1 in molecular weight distribution, ring-opening degree of uronic acid, galacturonic acid content, polydispersity, and the like.

**Biological Experiments**

The following tested the manufactured products from the perspectives of heparanase inhibition in vitro, resistance to cancer cell growth, resistance to cancer cell metastasis, and acute toxicity, respectively.

**Test Example 1.** Assay for In Vitro Inhibitory Activity Against Heparanase (Fondaparinux Method with Fondaparinux as Substrate)

The in vitro heparanase inhibitory activity of the products manufactured in the present application was tested with reference to the method for determining the heparanase inhibitory activity in vitro in CN105744940A. The results are shown in Table 4 below.

Table 4

| Sample No. | Inhibitory activity against heparanase ($IC_{50}$, ng/mL) |
|---|---|
| Sample A from Example 1 | 8.7 |
| Sample B from Example 2 | 8.9 |
| Sample C from Example 3 | 9.6 |
| Sample D from Example 4 | 5.2 |
| Sample E from Example 5 | 9.8 |
| Sample F from Example 6 | 9.4 |
| Sample G from Example 7 | 8.3 |
| Sample H from Example 8 | 6.7 |

(continued)

| Sample No. | Inhibitory activity against heparanase ($IC_{50}$, ng/mL) |
|---|---|
| Sample I from Example 9 | 9.9 |
| Sample J from Example 10 | 8.5 |
| Sample from Comparative Example 1 | 15.5 |

As shown in Table 4 above, the products of the present disclosure have excellent heparanase inhibitory activity.

**Test Example 2.** Isotope Method - Assay for Inhibitory Activity Against Heparanase

Endothelial cells were cultured with a culture medium containing $^{35}S$ isotope, and when they were confluent, the cells were removed, leaving extracellular matrix. The $^{35}S$-labeled endothelial cell extracellular matrix was coated on the surface of a 35 mm tissue culture dish and incubated with recombinant human heparanase (200 ng/mL) in the absence and presence of an inhibitory molecule (5 h, 37 °C, pH 6.0, final volume: 1 mL). The reaction mixture contained: 50 mm NaCl, 1 mm DTT, 1 mm $CaCl_2$, and 10 mm phosphate-citrate buffer, pH 6.0. To assess the degree of extracellular matrix degradation, the culture broths were collected and subjected to gel filtration on a Sepharose 6B column. Fractions (0.2 mL) were eluted with PBS and counted for radioactivity. The exclusion volume (Vo), elution volume (Ve), and total inclusion volume (Vt) were recorded, and the Kav value can be calculated, where Kav = (Ve - Vo)/(Vt - Vo), where for the Sepharose 6B column, the products in the elution volume corresponding to Kav at 0.5 to 0.8 were degradation fragments of extracellular matrix, corresponding to fractions 20-35. By setting the experimental conditions such that the heparanase activity corresponding to the radioactivity of fractions 20-35 in the absence of heparanase was defined as 0 and the heparanase activity corresponding to the radioactivity in the absence of an inhibitory molecule was defined as 100%, the heparanase inhibitory activities corresponding to the inhibitory products at different concentrations could be obtained. The $IC_{50}$ values of the different inhibitory products were obtained by calculation. The specific experimental results are shown in Table 5 below.

Table 5

| Sample No. | Inhibitory activity against heparanase ($IC_{50}$, ng/mL) |
|---|---|
| Sample A from Example 1 | 286.5 |
| Sample B from Example 2 | 299.3 |
| Sample C from Example 3 | 332.9 |
| Sample D from Example 4 | 190.3 |
| Sample E from Example 5 | 311.6 |
| Sample F from Example 6 | 296.1 |
| Sample G from Example 7 | 250.4 |
| Sample H from Example 8 | 229.2 |
| Sample I from Example 9 | 358.8 |
| Sample J from Example 10 | 288.7 |
| Sample from Comparative Example 1 | 534.4 |

It is well known to those skilled in the art that the isotope method-heparanase inhibitory activity assay method used in this test example has significant advantages over the fondaparinux method-heparanase inhibitory activity assay method in Test Example 1. The advantage of the method in this test example is that the heparan sulfate substrate labeled with an isotope is used instead of fondaparinux, and the isotope-labeled heparan sulfate substrate is closer to the heparanase substrate in the extracellular microenvironment of an organism, so that the heparanase inhibitory activity of the compound of the present disclosure in the in vivo environment can be better characterized.

As can be seen from the results in Table 5, the inhibitory activity of the products manufactured by the present application against heparanase was all significantly better than that of Comparative Sample 1.

**Test Example 3.** Anti-Tumor Efficacy in The Treatment of Subcutaneous Human Breast Cancer Xenograft Model MDA-MB-231 in BALB/c Nude Mice

Experimental materials: female BALB/c Nude mice (6-7 weeks old, purchased from Beijing Vital River Laboratory Animal Technology Co., Ltd., production license number: SCXK(Beijing)2021-0006), experimental samples (manufactured according to the methods of the examples and comparative examples of the present disclosure), purified water, normal saline (purchased from Guangdong Kelun Pharmaceutical Co., Ltd., lot No. G22121402A), MDA-MB-231 cells (purchased from American Type Culture Collection ATCC), DMEM culture medium (Gibco, Thermo Fisher Scientific (China) Co., Ltd.), fetal bovine serum (Gibco, Thermo Fisher Scientific (China) Co., Ltd.), horse serum (Gibco, Thermo Fisher Scientific (China) Co., Ltd.), trypsin (Gibco, Thermo Fisher Scientific (China) Co., Ltd.), and matrigel (Corning).

Experimental method: MDA-MB-231 cells were subjected to in vitro adherent culture, and the cells were collected when the cells were in the exponential growth phase. A subcutaneous mouse breast cancer tumor model was constructed by subcutaneously inoculating $1 \times 10^7$ MDA-MB-231 tumor cells into the right anterior scapula of each BALB/c nude mouse. When the mean volume of the tumor reached about 150 $mm^3$, the mice were randomly grouped according to the tumor volume, 10 mice/group.

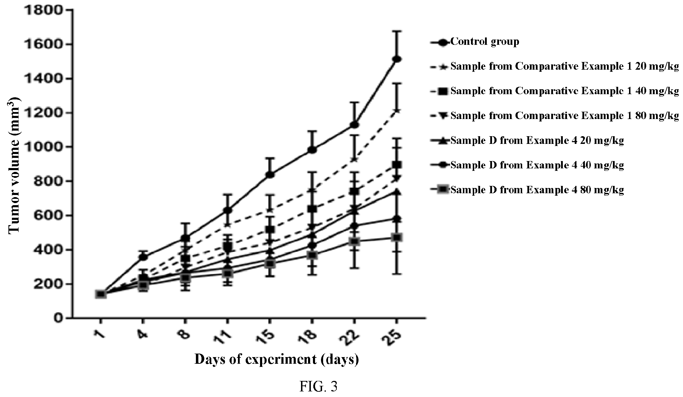

In the experiment, an experimental group and a control group were set up. The product solutions of the examples of the present disclosure and the sample solution in Comparative Example 1 were separately administered to the experimental group by subcutaneous injection. The experimental group was administered at doses of 20 mg/kg, 40 mg/kg, and 80 mg/kg, once daily. The control group was administered with an equal volume of normal saline by subcutaneous injection once daily. The time before administration was recorded as day D0, and the time of the first administration was recorded as D1. The tumor diameters were measured twice weekly with a vernier caliper, and the body weights of the mice were measured. The effect of the drug on tumor growth was examined by the obtained T/C% or tumor growth inhibition rate TGI (%) calculated according to the following formula, and the tumor growth curves are shown in FIG. 3.

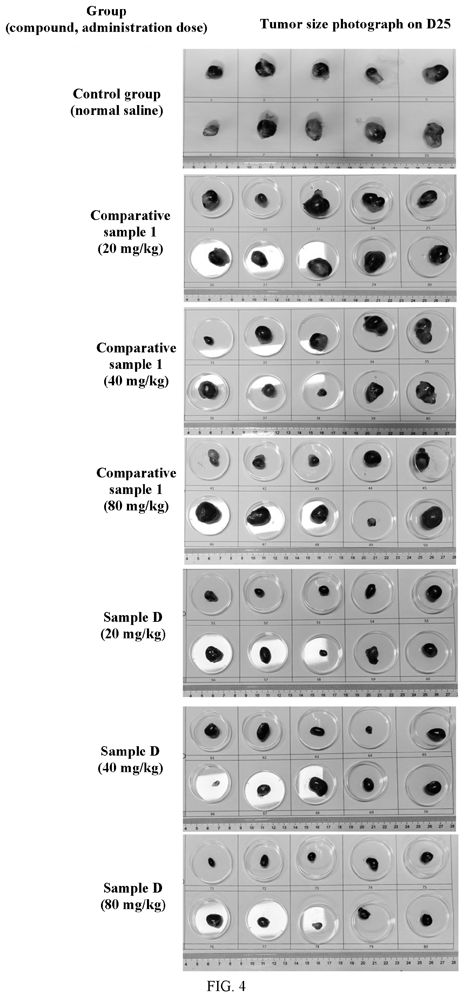

At the end of the experiment (the experiment ended on day D25), at the experiment endpoint, or when the tumor volume reached 1500 $mm^3$, the animals were sacrificed by $CO_2$ anesthesia and dissected to give the tumors, which were photographed, and the photographs of tumor size are shown in FIG. 4. The inhibitory effect of the drug on the tumor is expressed as tumor growth inhibition rate TGI. The experimental results are shown in Table 6 below. The tumor volume (V) was calculated as follows: $V = 1/2 \times a \times b^2$, where a and b represent long diameter and short diameter, respectively.

The TGI calculation formula is as follows: $TGI\% = [1 - (Ti - T0)/(Ci - C0)] \times 100\%$; Ti is the mean tumor volume of the treatment group at a particular time point, and T0 is the mean tumor volume of the treatment group at the beginning of the experiment (the day of grouping); Ci is the mean tumor volume of the control group at a particular time point, and C0 is the mean tumor volume of the control group at the beginning of the experiment (the day of grouping).

Table 6. Efficacy of test samples on subcutaneous xenograft tumor of human breast cancer xenograft cells MDA-MB-231

| Compound (Administration dose) | Mean tumor volume ($mm^3$) | | Mean mouse body weight (g) | | Mean weight of tumor on D25 (g) | Tumor inhibition rate on D25 (%) |
|---|---|---|---|---|---|---|
| | D0 | D25 | D0 | D25 | | |
| Control group (normal saline) | 140.00 | 1514.31 | 20.46 | 22.90 | 1.61 | N/A |
| Sample from Comparative Example 1 (20 mg/kg) | 140.43 | 1213.46 | 21.03 | 23.80 | 1.33 | 22% |
| Sample from Comparative Example 1 (40 mg/kg) | 140.49 | 897.51 | 21.11 | 23.45 | 0.93 | 45% |
| Sample from Comparative Example 1 (80 mg/kg) | 141.81 | 854.56 | 20.97 | 22.68 | 0.87 | 51% |
| Sample D from Example 4 (20 mg/kg) | 140.76 | 742.43 | 21.38 | 23.98 | 0.78 | 56% |
| Sample D from Example 4 (40 mg/kg) | 140.98 | 582.33 | 20.82 | 23.52 | 0.59 | 68% |
| Sample D from Example 4 (80 mg/kg) | 141.45 | 471.86 | 20.86 | 23.45 | 0.44 | 76% |

The results are shown in Table 6. The products of the present disclosure can significantly inhibit the growth of

subcutaneous tumors in nude mice bearing MDA-MB-231 human xenograft cells. The tumor inhibition rates at the three doses of 20 mg/kg, 40 mg/kg and 80 mg/kg were 56%, 68% and 76%, respectively, showing a dose-dependent relationship. Moreover, the tumor inhibition rate at each dose was all superior to that of the sample from Comparative Example 1 (the tumor inhibition rates at the corresponding three doses were 22%, 45% and 51%, respectively). The tumor-bearing mice were able to well tolerate all the above drugs, and no symptoms such as death and significant body weight loss were observed, indicating that the compound of the present disclosure have good safety.

**Test Example 4.** Anti-tumor Efficacy in The Treatment of Subcutaneous Pancreatic Cancer Xenograft Model Mia PaCa-2 in BALB/c Nude Mice

Experimental materials: female BALB/c Nude mice (6-7 weeks old, purchased from Beijing Vital River Laboratory Animal Technology Co., Ltd., production license number: SCXK(Beijing)2021-0006), experimental samples (manufactured according to the methods of the examples and comparative examples of the present disclosure), purified water, normal saline (purchased from Guangdong Kelun Pharmaceutical Co., Ltd., lot No. G22121402A), Mia PaCa-2 cells (purchased from Cell Bank of Chinese Academy of Sciences), DMEM culture medium (Gibco, Thermo Fisher Scientific (China) Co., Ltd.), fetal bovine serum (Gibco, Thermo Fisher Scientific (China) Co., Ltd.), horse serum (Gibco, Thermo Fisher Scientific (China) Co., Ltd.), trypsin (Gibco, Thermo Fisher Scientific (China) Co., Ltd.), and matrigel (Corning).

Experimental method: Mia PaCa-2 cells were subjected to in vitro adherent culture, and the cells were collected when the cells were in the exponential growth phase. A subcutaneous mouse pancreatic cancer tumor model was constructed by subcutaneously inoculating $5 \times 10^6$ Mia PaCa-2 cells into the right anterior scapula of each BALB/c nude mouse. When the mean volume of the tumor reached about 150 $mm^3$, the mice were randomly grouped according to the tumor volume, 9 mice/group.

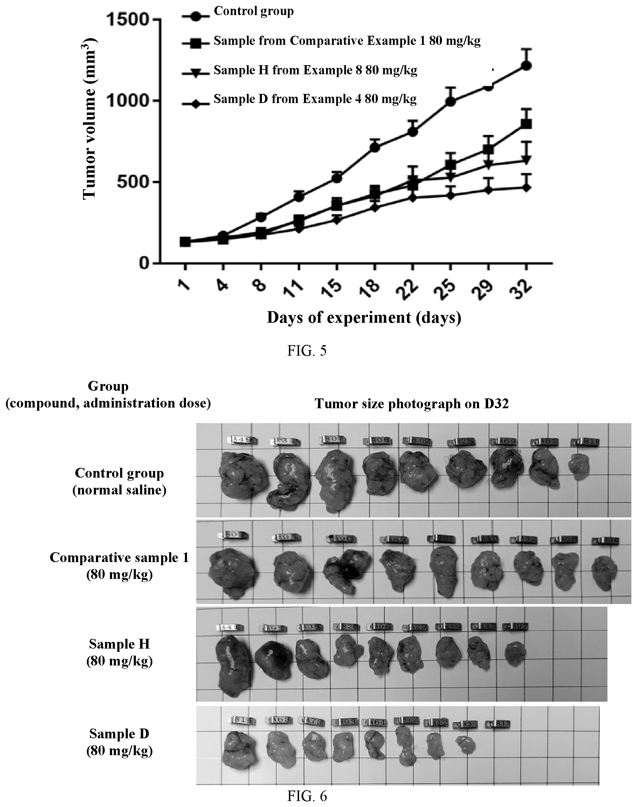

The sample solutions obtained in the examples of the present disclosure and the sample solution in Comparative Example 1 were separately administered to the treatment group and the comparative experimental group by subcutaneous injection at a dose of 80 mg/kg 5 days per week, and the control group was administered by subcutaneous injection with an equal volume of normal saline 5 days per week. The time before administration was recorded as day D0, and the time of the first administration was recorded as D1. The tumor diameters were measured twice weekly with a vernier caliper, and the body weights of the mice were measured. The effect of the drug on tumor growth was examined by the obtained T/C% or tumor growth inhibition rate TGI (%) calculated according to the following formula, and the tumor growth curves are shown in FIG. 5.

At the end of the experiment (the experiment ended on day D32), at the experiment endpoint, or when the tumor volume reached 1500 $mm^3$, the animals were sacrificed by $CO_2$ anesthesia and dissected to give the tumors, which were photographed, and the tumor sizes are shown in FIG. 6. The inhibitory effect of the drug on the tumor is expressed as tumor growth inhibition rate TGI. The experimental results are shown in Table 7 below.

The tumor volume (V) was calculated as follows: $V = 1/2 \times a \times b^2$, where a and b represent long diameter and short diameter, respectively.

The TGI calculation formula is as follows: $TGI\% = [1 - (Ti - T0)/(Ci - C0)] \times 100\%$; Ti is the mean tumor volume of the treatment group at a particular time point, and T0 is the mean tumor volume of the treatment group at the beginning of the experiment (the day of grouping); Ci is the mean tumor volume of the control group at a particular time point, and C0 is the mean tumor volume of the control group at the beginning of the experiment (the day of grouping).

Table 7. Efficacy of test samples on subcutaneous xenograft tumor of human pancreatic cancer xenograft cells Mia PaCa-2

| Group (compound, administration dose) | Mean tumor volume ($mm^3$) | | Mean mouse body weight (g) | | Mean weight of tumor on D32 (g) | Tumor inhibition rate on D32 (%) |
|---|---|---|---|---|---|---|
| | D0 | D32 | D0 | D32 | | |
| Control group (normal saline) | 132.77 | 1218.58 | 21.53 | 24.23 | 1.14 | N/A |
| Sample from Comparative Example 1 (80 mg/kg) | 132.72 | 859 | 21.37 | 24.02 | 0.79 | 33% |
| Sample H from Example 8 (80 mg/kg) | 132.79 | 632.84 | 21.47 | 24.79 | 0.54 | 54% |
| Sample D from Example 4 (80 mg/kg) | 132.72 | 468 | 21.52 | 25.24 | 0.37 | 69% |

The results are shown in Table 7. The compounds of the present disclosure had significant inhibitory effects on the growth of subcutaneous tumors of Mia PaCa-2 human xenograft cells in nude mice, and the tumor inhibition rates were 54% and 69%. The sample from Comparative Example 1 had a tumor inhibition rate of 33% on Mia PaCa-2 subcutaneous xenograft tumors, indicating no significant therapeutic effect. The tumor-bearing mice were able to well tolerate all the above drugs, and no symptoms such as death and significant body weight loss were observed, indicating that the compound of the present disclosure have good safety.

**Test Example 5.** Anti-tumor Efficacy in The Treatment of Subcutaneous Murine Myeloma Model MPC-11

Experimental materials: female BALB/c Nude mice (6-7 weeks old, purchased from Beijing Vital River Laboratory Animal Technology Co., Ltd., production license number: SCXK(Beijing)2021-0006), experimental compounds (manufactured according to the methods of the examples and comparative examples of the present disclosure), purified water, normal saline (purchased from Guangdong Kelun Pharmaceutical Co., Ltd., lot No. G22121402A), MPC-11 murine myeloma cells (purchased from American Type Culture Collection ATCC), DMEM culture medium (Gibco, Thermo Fisher Scientific (China) Co., Ltd.), fetal bovine serum (Gibco, Thermo Fisher Scientific (China) Co., Ltd.), horse serum (Gibco, Thermo Fisher Scientific (China) Co., Ltd.), trypsin (Gibco, Thermo Fisher Scientific (China) Co., Ltd.), and matrigel (Corning).

Experimental method: MPC-11 murine myeloma cells were cultured in suspension in vitro and collected when the cells were in the exponential growth phase. A subcutaneous mouse myeloma tumor model was constructed by subcutaneously inoculating $5 \times 10^5$ MPC-11 murine myeloma cells into the right back of each BALB/c nude mouse. The experimental mice were randomly grouped, 8 mice/group.

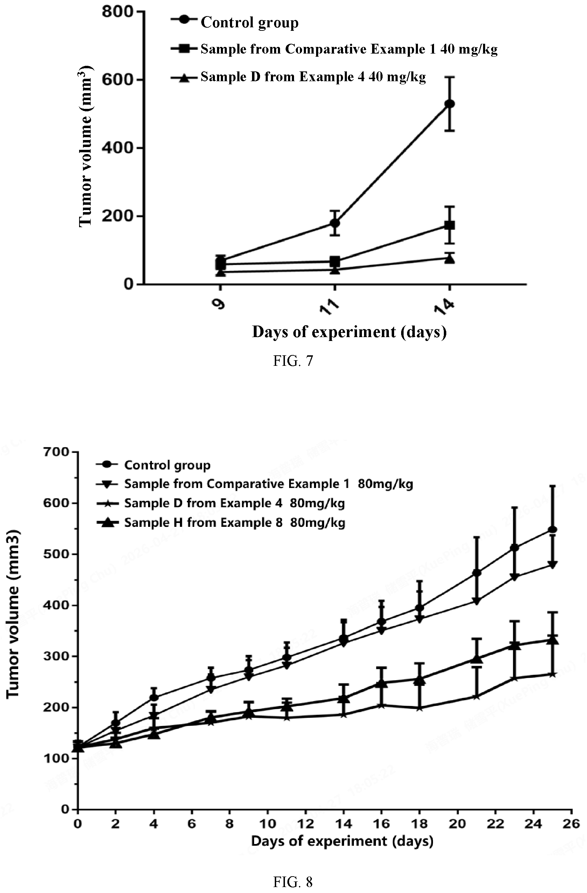

From day 2 after inoculation, the sample solution of the present disclosure and the sample solution in Comparative Example 1 were separately administered to the treatment group and the comparative experimental group by subcutaneous injection at a dose of 40 mg/kg once daily, and the control group was administered by subcutaneous injection with an equal volume of normal saline once daily. The body weight and tumor diameters of the mice in each group were measured on days 9, 11, and 14 after inoculation. The effect of the drug on tumor growth was examined by the obtained T/C% or tumor growth inhibition rate TGI (%) calculated according to the following formula, and the tumor growth curves are shown in FIG. 7. Based on the tumor growth, the experiment ended on day 15 after inoculation. The animals were euthanized and the tumors were collected.

TGI: $TGI\% = [1 - (Ti - T0)/(Ci - C0)] \times 100\%$; Ti is the mean tumor volume of the treatment group at a particular time point, and T0 is the mean tumor volume of the treatment group on the day of grouping; Ci is the mean tumor volume of the control group at a particular time point, and C0 is the mean tumor volume of the control group on the day of grouping. Due to the experimental setting, in this experiment, both $T_0$ and $C_0$ were 0. The experimental results are shown in Table 8 below.

Table 8. Efficacy of test samples on subcutaneous tumor of murine myeloma cells MPC-11

| Group (compound, administration dose) | Mean tumor volume ($mm^3$) | | | Tumor inhibition rate on D14 (%) |
|---|---|---|---|---|
| | D9 | D11 | D14 | |
| Control group | 70.22 | 180.91 | 530.23 | N/A |
| Sample from Comparative Example 1 (40 mg/kg) | 59.02 | 67.54 | 174.28 | 67% |
| Sample D from Example 4 (40 mg/kg) | 36.87 | 43.35 | 78.66 | 85% |

The results are shown in Table 8 above. The samples of the present disclosure can significantly inhibit the growth of MPC-11 murine myeloma subcutaneous tumors, and the tumor inhibition rates of the samples of the present disclosure were significantly superior to those of the control group and the sample from Comparative Example 1. **Test Example 6.** Anti-tumor efficacy in the treatment of subcutaneous human Mesothelioma xenograft model MSTO-211H in BALB/c nude mice Experimental materials: female BALB/c Nude mice (6-7 weeks old, purchased from Beijing Vital River Laboratory Animal Technology Co., Ltd., production license number: SCXK(Beijing)2021-0006), experimental compounds (manufactured according to the methods of the examples and comparative examples of the present disclosure), purified water, normal saline (purchased from Guangdong Kelun Pharmaceutical Co., Ltd., lot No. G22121402A), MSTO-211H cells (purchased from American Type Culture Collection ATCC), DMEM culture medium (Gibco, Thermo Fisher Scientific (China) Co., Ltd.), fetal bovine serum (Gibco, Thermo Fisher Scientific (China) Co., Ltd.), horse serum (Gibco, Thermo Fisher Scientific (China) Co., Ltd.), trypsin (Gibco, Thermo Fisher Scientific (China) Co., Ltd.), and matrigel (Corning).

Experimental method: MSTO-211H human mesothelioma cells were subjected to in vitro adherent culture, and the cells were collected when the cells were in the exponential growth phase. A subcutaneous mesothelioma tumor model was constructed by subcutaneously inoculating $5 \times 10^6$ MSTO-211H tumor cells into the right back of each BALB/c nude

mice. When the tumors grew to an average volume of about 150 $mm^3$, the mice were randomly grouped according to the tumor volume, 10 mice/group.

In the experiment, a treatment group, a comparative experimental group, and a control group were set up. The sample solutions of the examples of the present disclosure and the sample solution in Comparative Example 1 were separately administered to the treatment group and the comparative experimental group by subcutaneous injection. The treatment group and the comparative experimental group were administered at a dose of 80 mg/kg once daily. The control group was administered with an equal volume of normal saline by subcutaneous injection once daily. The time before administration was recorded as day D0, and the time of the first administration was recorded as D1. The tumor diameters were measured twice weekly with a vernier caliper, and the body weights of the mice were measured. The effect of the drug on tumor growth was examined by the obtained tumor growth inhibition rate TGI (%) calculated according to the following formula, and the tumor growth curves are shown in FIG. 8. The experiment ended on day 25. The animals were sacrificed by $CO_2$ anesthesia and dissected to give the tumors, which were photographed. The inhibitory effect of the drug on the tumor is expressed as tumor growth inhibition rate TGI. The experimental results are shown in Table 9 below.

The tumor volume (V) was calculated as follows: $V = 1/2 \times a \times b^2$, where a and b represent long diameter and short diameter, respectively.

The TGI calculation formula is as follows: $TGI\% = [1 - (Ti - T0)/(Ci - C0)] \times 100\%$; Ti is the mean tumor volume of the treatment group at a particular time point, and T0 is the mean tumor volume of the treatment group at the beginning of the experiment (the day of grouping); Ci is the mean tumor volume of the control group at a particular time point, and C0 is the mean tumor volume of the control group at the beginning of the experiment (the day of grouping).

Table 9. Efficacy of test samples on subcutaneous xenograft tumor of human mesothelioma xenograft cells MSTO-211H

| Group (compound, administration dose) | Mean tumor volume ($mm^3$) | | Mean mouse body weight (g) | | Mean weight of tumor on D25 (g) | Tumor inhibition rate on D25 (%) |
|---|---|---|---|---|---|---|
| | D0 | D25 | D0 | D25 | | |
| Control group (normal saline) | 122.78 | 548.89 | 20.34 | 22.22 | 0.55 | N/A |
| Sample from Comparative Example 1 (80 mg/kg) | 121.82 | 479.72 | 20.06 | 22.29 | 0.46 | 16% |
| Sample D from Example 4 (80 mg/kg) | 121.53 | 265.50 | 20.01 | 23.21 | 0.23 | 66% |
| Sample H from Example 8 (80 mg/kg) | 122.54 | 332.87 | 20.24 | 22.93 | 0.32 | 50% |

The results are shown in Table 9. The compounds of the present disclosure had significant inhibitory effects on the growth of subcutaneous tumors of MSTO-211H human mesothelioma xenograft cells in nude mice. At the dose of 80 mg/kg, the tumor inhibition rates of sample D from Example 4 and sample H from Example 8 were 66% and 50%, respectively. The sample from Comparative Example 1 had a tumor inhibition rate of 16% on MSTO-211H subcutaneous xenograft tumors, indicating no significant therapeutic effect. The tumor-bearing mice were able to well tolerate all the above drugs, and no symptoms such as death and significant body weight loss were observed, indicating that the compound of the present disclosure have good safety. The results show that the compounds of the present disclosure have better tumor inhibitory activity than that of the sample from Comparative Example 1.

**Test Example 7.** Pharmacokinetic Experiment

The purpose of this experiment was to study the single subcutaneous injection administration of the compound solution of the present disclosure and the control sample solution to SD rats, to detect the corresponding components in the plasma, and to evaluate their pharmacokinetic (PK) profiles in SD rats. The inventors of the present disclosure have found through stability experiments with liver microsomes of different species that: the metabolism of the compounds of the present disclosure in the liver microsomes of different species is substantially similar, so SD rats were selected for this PK study.

Experimental materials: SD rats (weighing 180-220 g, purchased from Beijing Vital River Laboratory Animal Technology Co., Ltd., production license No. SCXK(Beijing)2021-0006), experimental compounds (manufactured according to the methods of the examples and the comparative examples of the present disclosure), and purified water.

Experimental method: the compounds obtained by the present disclosure and the control sample were all heparin glycosaminoglycan derivatives. Terminal aldehyde groups thereof could be linked to tyramide by a reductive amination reaction, and $^{125}I$ ion was linked to the tyramide under the condition of an oxidant, so that [$^{125}I$] labeling of the

heparin glycosaminoglycan derivatives was achieved. An animal test was performed after the labeled samples passed the quality control of physicochemical properties and biological activity.

SD rats were randomly grouped (6 rats per group, half male and half female), given ad libitum access to water during the assay, fasted for 12 or more hours before administration, and given food 4 hours after administration. The administration was performed by subcutaneous injection, and each group of SD rats was given a solution of the experimental compound in normal saline at a dose of 20 mg/kg (on a compound basis). Before the administration test, the animals were intragastrically given 1% KI to block the thyroid. The drinking water for the animals during the test was a 0.01% KI solution.

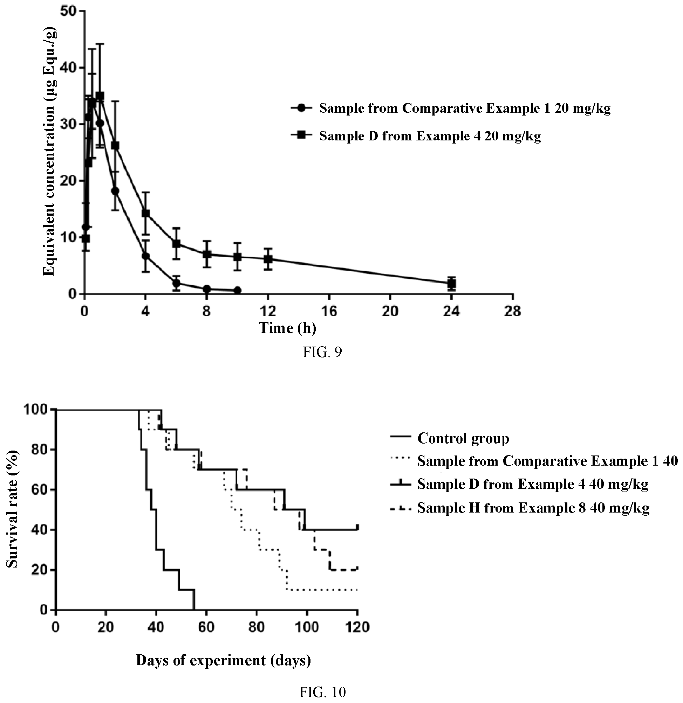

Whole blood was collected at 0 min before administration and at 5 min, 15 min, 30 min, 1 h, 2 h, 4 h, 6 h, 8 h, 10 h, 12 h, 24 h, 48 h, and 72 h after administration. The blood was centrifuged within 60 min after blood collection to separate plasma (centrifuged at 8,000 rpm at 2-8 °C for 5 min). The total radioactivity was detected using a $\gamma$ counter. The concentrations of radioactive substances in the plasma were obtained, and the data were analyzed using a non-compartmental model and DAS 3.2.8 software to evaluate their pharmacokinetic (PK) profiles in SD rats and calculate pharmacokinetic parameters. The data are shown in Table 10, and the pharmacokinetic curves are shown in FIG. 9. Since the results measured in this experiment were radioactivity data, the pharmacokinetic concentration data were obtained by conversion. Therefore, the data were expressed in μg Equ./g as equivalent concentration values after the calculation of radioactivity data.

Table 10. Pharmacokinetic parameters in rat plasma after subcutaneous injection of compound of the present disclosure and sample from Comparative Example 1

| Parameter \ Compound | Sample D from Example 4 | Sample of Comparative Example 1 |
|---|---|---|
| $T_{1/2}$ (h) | 7.73 | 1.18 |
| $T_{max}$ (h) | 1.00 | 0.5 |
| $C_{max}$ (μg Equ./g) | 36.21 | 34.41 |
| $AUC_{last}$ (h*μg Equ./g) | 217.21 | 90.38 |

The results in Table 10 show that the compound of the present disclosure had significantly improved pharmacokinetic properties, and in particular, AUC and $T_{1/2}$ for administration of the compound of the present disclosure were both very significantly improved, indicating that the compound of the present disclosure have good bioavailability compared with the sample from Comparative Example 1, and is beneficial for preparing into a sustained-release preparation or long-acting preparation for administration once a day or at a longer administration interval, which can greatly improve patient compliance and the convenience of administration for healthcare providers.

**Test Example 8.** Characterization of Bleeding Risk of Sample by Determining Activated Partial Thromboplastin Time (aPTT)

Activated partial thromboplastin time is also a relatively sensitive screening test for the coagulation system. The activated partial thromboplastin time mainly reflects whether the exogenous coagulation is normal.

After the aPTT reagent is added to the anticoagulated plasma, the endogenous coagulation pathway is initiated, such that XI is activated to XIa, and part of glycosaminoglycan substances may act on the coagulation process, thereby prolonging the aPTT and leading to an increased risk of bleeding. Therefore, the determination of aPTT is used to detect the anticoagulation activity of the sample of the present disclosure to characterize the possible risk of bleeding. The specific steps were as follows: rat plasma (purchased from Guangzhou Hongquan Biotechnology Co., Ltd.), beagle plasma (purchased from Guangzhou Hongquan Biotechnology Co., Ltd.), cynomolgus monkey plasma (purchased from Guangzhou Hongquan Biotechnology Co., Ltd.), and human plasma (purchased from Beijing Huizhiheyuan Biotechnology Co., Ltd., under trade name IPHASE) were each taken and anticoagulated with 3.2% sodium citrate. The plasma was all mixed with multiple samples of the same species at 100 mL/vial, and 24 tubes of plasma (0.6 mL per tube) for each species were collected and stored at 4 °C for later use. The test compounds (the compounds of the examples of the present disclosure and the sample from Comparative Example 1) were dissolved and diluted with normal saline, and test sample stock solutions at 4 concentration points of 2.63 mg/mL, 1.32 mg/mL, 0.66 mg/mL, and 0.33 mg/mL were formulated in sequence. The test sample stock solutions at each concentration were subjected to a 100-fold dilution to obtain test sample solutions (i.e., 495 μL of plasma was taken, and 5 μL of a test sample stock solution was added to obtain the test sample solution).

Subsequently, the aPTT of each sample was measured using an automatic coagulation analyzer (purchased from Stago, model STA-R), and the experiment was repeated 3 times for each sample. The results are detailed in Table 11 below. The aPTT values when adding equal amounts of sample D from Example 4 and the sample from Comparative Example 1 were compared to evaluate the risk of bleeding of the samples; the smaller the aPTT value, the lower the risk of bleeding, and the higher the safety.

Table 11. aPTT values of samples in different concentrations and different plasma

| Group (experimental compound, concentration) | SD rat plasma aPTT (s) | Beagle plasma aPTT (s) | Cynomolgus monkey plasma aPTT (s) | Human plasma aPTT (s) |
|---|---|---|---|---|
| Control group (normal saline) | 22.1 | 24.0 | 23.6 | 29.6 |
| Sample D from Example 4 (3.3 μg/mL) | 23.8 | 29.7 | 25.9 | 33.0 |
| Sample D from Example 4 (6.6 μg/mL) | 27.1 | 36.1 | 33.5 | 39.5 |
| Sample D from Example 4 (13.2 μg/mL) | 33.3 | 50.0 | 45.4 | 51.6 |
| Sample D from Example 4 (26.3 μg/mL) | 41.0 | 73.9 | 70.4 | 83.7 |
| Sample from Comparative Example 1 (3.3 μg/mL) | 24.1 | 30.4 | 26.5 | 34.1 |
| Sample from Comparative Example 1 (6.6 μg/mL) | 27.9 | 37.8 | 35.2 | 41.6 |
| Sample from Comparative Example 1 (13.2 μg/mL) | 35.0 | 53.8 | 48.9 | 55.6 |
| Sample from Comparative Example 1 (26.3 μg/mL) | 43.8 | 81.2 | 77.7 | 92.4 |

As shown in the results in Table 11 above, the aPTT values of the compounds of the present disclosure at each concentration were all smaller than that of the sample from Comparative Example 1, which performed uniformly in various species. This indicates that the compounds of the present disclosure have a lower risk of bleeding and higher safety than the sample from Comparative Example 1. In conclusion, the compounds of the present disclosure have lower bleeding risk, higher safety, and good drug safety.

**Test Example 9.** Animal Experiment of In Situ 4T1 Breast Cancer with Lung Metastases

4T1 cells were cultured in suspension in vitro under the conditions of an RPMI 1640 culture medium plus 10% heat-inactivated fetal bovine serum at 37 °C with 5% $CO_2$. Subculturing was performed three times a week. When the cells were in the exponential growth phase, the cells were collected and counted, and 50 μL of the cell suspension containing $1 \times 10^5$ tumor cells (the cells were suspended in RPMI 1640 culture medium without fetal bovine serum) was inoculated into the fourth abdominal fat pad of female BALB/c mice aged 6-8 weeks and weighing 18-22 g. The mice were randomly grouped according to the body weight and the order of tumor inoculation, with 10 mice in each group. The compounds of the present disclosure and the sample from Comparative Example 1 were administered to the experimental group by subcutaneous injection once a day on the second day after inoculation at a dose of 40 mg/kg, and the control group was given an equal amount of normal saline. The in-situ tumor was surgically removed on day 12 after tumor inoculation, and the administration was not performed on that day. The administration was continued in the above manner the next day after the operation.

Mouse survival time: each mouse was euthanized at the experimental endpoint (death or body weight loss greater than 20%) and the survival time was recorded. Kaplan-Meier survival curves were plotted, and the results are shown in FIG. 10. The median survival time and the percentage increase of median survival time were calculated. The specific results are shown in Table 12 below. The formula for calculating the percentage increase of median survival time was as follows:

Percentage increase of median survival time (%) = (median survival time of administration group - median survival time of control group)/median survival time of control group $\times$ 100%.

Table 12

| Group (compound, dose) / Indicator | Control group (normal saline) | Sample from Comparative Example 1 40 mg/kg | Sample D from Example 4 40 mg/kg | Sample H from Example 8 40 mg/kg |
|---|---|---|---|---|
| Median survival time (day) | 39 | 72 | 95 | 92 |
| Percentage increase of median survival time (%) | NA | 85% | 146% | 136% |

As shown in Table 12 above, the compounds of the present disclosure were able to significantly prolong the median survival time of the experimental subjects; at the dose of 40 mg/kg, the median survival time of the compound groups of the present disclosure relative to the sample group of Comparative Example 1 were significantly prolonged, by 146% and 136%, respectively, for the sample D group of Example 4 and the sample H group of Example 8. The sample from Comparative Example 1 was able to prolong the median survival time of model mice by 85% compared with the control group, which was significantly lower than that of the compounds of the present disclosure. The above indicates that the compounds of the present disclosure have stronger activity in inhibiting tumor metastasis and/or tumor growth compared with the sample from Comparative Example 1, and the samples of the present disclosure can greatly prolong the survival time of the experimental subjects.

The embodiments of the present disclosure have been described above. However, the embodiments described above are not intended to limit the present disclosure. Any modification, equivalent replacement, improvement, and the like made without departing from the spirit and principle of the present disclosure shall fall within the claimed scope of the present disclosure.

## Claims

1. A chemically modified glycosaminoglycan, comprising a structural unit of formula (I), a structural unit of formula (II), a structural unit of formula (III), and a structural unit of formula (IV):

formula (I), formula (II), formula (III), formula (IV);

wherein:

$R^{1m}$, $R^{1n}$, and $R^{1q}$, at each occurrence, are each independently selected from H, $-SO_3^-$, and $-(C{=}O)CH_3$; and preferably, $R^{1m}$ and $R^{1n}$, at each occurrence, are $-SO_3^-$ or $-(C{=}O)CH_3$;
$R^{2m}$, $R^{2n}$, $R^{2q}$, and $R^{2x}$, at each occurrence, are each independently selected from H and $-SO_3^-$;
$R^{3n}$, at each occurrence, is each independently selected from H and $-SO_3^-$;
$R^{4m}$ and $R^{4n}$, at each occurrence, are each independently selected from H and $-SO_3^-$, and
the chemically modified glycosaminoglycan has a weight-average molecular weight of 10,000 Da to 15,000 Da, preferably 10,500 Da to 14,000 Da.

2. The chemically modified glycosaminoglycan as claimed in claim 1, wherein the chemically modified glycosaminoglycan has a content of galacturonic acid of 0.10%-2.00%, preferably 0.3%-1.90%;

and/or, the chemically modified glycosaminoglycan has a ring-opening degree of uronic acid of 45%-75%;
and/or, the the chemically modified glycosaminoglycan has a sulfo-carboxyl ratio ($SO_3^-/COO^-$) of 0.7-1.5;

and/or, the chemically modified glycosaminoglycan has a carboxyl increment of 1.20-2.00;
and/or, the chemically modified glycosaminoglycan has a polydispersity index of 1.00-1.24, preferably 1.05-1.22, and further preferably 1.10-1.20;
and/or, the chemically modified glycosaminoglycan has a epoxy degree of uronic acid of less than 10%, preferably 0-5.0%;
and/or, the molecular weight distribution of the chemically modified glycosaminoglycan is as follows:

| | |
|---|---|
| greater than 19,000 Da | 0-20% |
| 6,000-19,000 Da | 73%-90% |
| less than 6,000 Da | 2%-15%; |

Preferably, the molecular weight distribution of the chemically modified glycosaminoglycan is as follows:

| | |
|---|---|
| greater than 19,000 Da | 0-18% |
| 6,000-19,000 Da | 73%-90% |
| less than 6,000 Da | 3%-15%. |

**3.** The chemically modified glycosaminoglycan as claimed in claim 1 or 2, wherein the chemically modified glycosaminoglycan further comprises a structural unit of formula (V):

formula (V)wherein:

$R^{2t}$ and $R^{3t}$, at each occurrence, are each independently selected from H and $-SO_3^-$;
and/or a structural unit of formula (VI):

formula (VI)

wherein:

$R^{2p}$, at each occurrence, is each independently selected from H and $-SO_3^-$;
and/or a structural unit of formula (VII):

formula (VII)

wherein:

$R^{2r}$ and $R^{3r}$, at each occurrence, are each independently selected from H and $-SO_3^-$;
and/or a structural unit of formula (VIII):

formula (VIII)

wherein:

$R^{1s}$, at each occurrence, is each independently selected from H, $-SO_3^-$, and -(C=O)$CH_3$, preferably $-SO_3^-$ or - (C=O)$CH_3$;
$R^{2s}$ and $R^{4s}$, at each occurrence, are each independently selected from H and $-SO_3^-$;
$R^{3s}$, at each occurrence, is $-SO_3^-$.

**4.** A method for preparing the chemically modified glycosaminoglycan as claimed in any one of claims 1-3, comprising the following steps:

a) epoxidizing C2, C3 of the uronic acid residue in the starting material glycosaminoglycan, preferably in the presence of a base (such as sodium hydroxide or potassium hydroxide);
b) hydrolysing the epoxidation product obtained in step a) to perform ring opening, preferably under a neutral condition;
c) under a condition effective to convert adjacent diols (vicinal diols) and optionally adjacent OH/$NH_2$ into dialdehydes, oxidizing 10%-100% (preferably 25%-100%) 2-O- and optionally 2-N-, 3-O-unsulfated residues of the glycosaminoglycan;
wherein the oxidation is performed in the presence of an oxidant selected from one of periodic acid and periodate (such as sodium periodate, or potassium periodate, etc.); and
d) under a condition effective to convert the dialdehydes into carboxyl groups and without nitrogen protection, further oxidizing the product obtained in step c),
wherein the further oxidation is preferably performed by a chlorite (such as sodium chlorite, magnesium chlorite $Mg(ClO_2)_2$, or barium chlorite $Ba(ClO_2)_2$, etc.);
e) separating and purifying the product of step d) by an anion exchange purification system to obtain the chemically modified glycosaminoglycan.

**5.** The method as claimed in claim 4, wherein in step a), the ratio of the molar amount of the base to the mass of the starting material glycosaminoglycan is 3.0 mmol/g to 15.0 mmol/g or 5.0 mmol/g to 12.0 mmol/g;

and/or, in step a), the reaction temperature is 55-75 °C;
and/or, in step a), the reaction time is 80 min to 800 min;
and/or, in step b), the reaction temperature is 60-85 °C;
and/or, in step b), the reaction time is 20-150 h or 36-96 h;
and/or, in step c), the ratio of the molar amount of the oxidant to the mass of the starting material glycosaminoglycan is 0.1 mmol/g to 10.0 mmol/g or 1.0 mmol/g to 5.0 mmol/g;
and/or, in step c), the reaction temperature is 0-20 °C;
and/or, in step c), the reaction time is 5-50 h or 10-30 h;
and/or, in step d), the ratio of the molar amount of chlorite to the mass of the starting material glycosaminoglycan is 3.0-20.0 mmol/g or 6.0-13.0 mmol/g;
and/or, in step d), the reaction temperature is 0-15 °C;
and/or, in step d), the reaction pH is 3.0-5.0;
and/or, in step d), the reaction time is 5-100 h or 12-36 h;
and/or, in step e), after separation and purification, the obtained chemically modified glycosaminoglycan has a polydispersity index of 1.00-1.24, e.g., 1.10-1.20;
and/or, in step e), after separation and purification, the obtained chemically modified glycosaminoglycan has a weight-average molecular weight of 10,000 Da to 15,000 Da, preferably 10,500 Da to 14,000 Da;

and/or, in step e), after separation and purification, the molecular weight distribution of the obtained chemically modified glycosaminoglycan is as follows:

| | |
|---|---|
| greater than 19,000 Da | 0-20% |
| 6,000-19,000 Da | 73%-90% |
| less than 6,000 Da | 2%-15% |

**6.** A chemically modified glycosaminoglycan obtained by the method as claimed in claim 4 or 5.

**7.** A pharmaceutical composition, comprising a prophylactically or therapeutically effective amount of the chemically modified glycosaminoglycan as claimed in any one of claims 1-3 and 6, and one or more pharmaceutically acceptable carriers.

**8.** The pharmaceutical composition as claimed in claim 7, wherein the pharmaceutical composition is in a form of a solid, semi-solid, liquid, or gas preparation.

**9.** The pharmaceutical composition as claimed in claim 7 or 8, wherein the pharmaceutical composition further comprises one or more additional therapeutic agents.

**10.** Use of the chemically modified glycosaminoglycan as claimed in any one of claims 1-3 and 6 or the pharmaceutical composition as claimed in any one of claims 7-9 for the manufacture of a medicament for the inhibition of tumor growth and/or metastasis, or use of the chemically modified glycosaminoglycan as claimed in any one of claims 1-3 and 6 or the pharmaceutical composition as claimed in any one of claims 7-9 for the manufacture of a medicament for the treatment and/or prevention of a tumor/cancer,
wherein preferably, the tumor is a solid tumor, a hematological tumor, or a soft tissue tumor, such as breast cancer, pancreatic cancer, bladder cancer, prostate cancer, colon cancer, gastric cancer, or lung cancer.

24.00
22.00
20.00
18.00
16.00
14.00
12.00
10.00
8.00
6.00
4.00
2.00
0.00
µRIU
9407
Intermediate 4j
Sample J
0.00
0.50
1.00
1.50
2.00
2.50
3.00
3.50
4.00
minute

FIG. 1

2a
Peak 1: C-H correlation signal peak at the C5 position of galacturonic acid
Peak 2: C-H correlation characteristic signal peak of acetyl group in N-acetylglucosamine
F1 [ppm]
70
80
90
100
5
4
3
F2 [ppm]

2b
Peak 1: C-H correlation signal peak at the C5 position of galacturonic acid
Peak 2: C-H correlation characteristic signal peak of acetyl group in N-acetylglucosamine
F1 [ppm]
70
80
90
100
5
4
3
F2 [ppm]
2c
Peak 1: C-H correlation signal peak at the C5 position of galacturonic acid
Peak 2: C-H correlation characteristic signal peak of acetyl group in N-acetylglucosamine
F1 [ppm]
70
80
90
100
5
4
3
F2 [ppm]


FIG. 2

1800
1600
1400
1200
1000
800
600
400
200
0
Tumor volume (mm3)
1
4
8
11
15
18
22
25
Days of experiment (days)
Control group
Sample from Comparative Example 1 20 mg/kg
Sample from Comparative Example 1 40 mg/kg
Sample from Comparative Example 1 80 mg/kg
Sample D from Example 4 20 mg/kg
Sample D from Example 4 40 mg/kg
Sample D from Example 4 80 mg/kg

FIG. 3

Group
(compound, administration dose)
Tumor size photograph on D25
Control group
(normal saline)
Comparative
sample 1
(20 mg/kg)
Comparative
sample 1
(40 mg/kg)
Comparative
sample 1
(80 mg/kg)
Sample D
(20 mg/kg)
Sample D
(40 mg/kg)
Sample D
(80 mg/kg)

FIG. 4

Control group
Sample from Comparative Example 1 80 mg/kg
Sample H from Example 8 80 mg/kg
Sample D from Example 4 80 mg/kg
Tumor volume (mm³)
1500
1000
500
0
1
4
8
11
15
18
22
25
29
32
Days of experiment (days)

FIG. 5

Group
(compound, administration dose)
Tumor size photograph on D32
Control group
(normal saline)
Comparative sample 1
(80 mg/kg)
Sample H
(80 mg/kg)
Sample D
(80 mg/kg)

FIG. 6

Control group
Sample from Comparative Example 1 40 mg/kg
Sample D from Example 4 40 mg/kg
Tumor volume (mm3)
800
600
400
200
0
9
11
14
Days of experiment (days)

FIG. 7

Control group
Sample from Comparative Example 1 80mg/kg
Sample D from Example 4 80mg/kg
Sample H from Example 8 80mg/kg
Tumor volume (mm3)
700
600
500
400
300
200
100
0
0 2 4 6 8 10 12 14 16 18 20 22 24 26
Days of experiment (days)

FIG. 8

Equivalent concentration (μg Equ./g)
50
40
30
20
10
0
0
4
8
12
16
20
24
28
Time (h)
Sample from Comparative Example 1 20 mg/kg
Sample D from Example 4 20 mg/kg

FIG. 9

Survival rate (%)
100
80
60
40
20
0
0
20
40
60
80
100
120
Days of experiment (days)
Control group
Sample from Comparative Example 1 40
Sample D from Example 4 40 mg/kg
Sample H from Example 8 40 mg/kg

FIG. 10

**INTERNATIONAL SEARCH REPORT**

International application No. **PCT/CN2024/130179**

**A. CLASSIFICATION OF SUBJECT MATTER**

C08B37/00(2006.01)i; C08B37/10(2006.01)i; C08L5/00(2006.01)i; A61K31/737(2006.01)i; A61P35/04(2006.01)i

According to International Patent Classification (IPC) or to both national classification and IPC

**B. FIELDS SEARCHED**

Minimum documentation searched (classification system followed by classification symbols)

IPC: C08B37/-, C08L5/-, A61K31/-, A61P35/-

Documentation searched other than minimum documentation to the extent that such documents are included in the fields searched

Electronic data base consulted during the international search (name of data base and, where practicable, search terms used)

CNTXT, ENTXT, ENTXTC, DWPI, VEN, VCN, WPABS, WPABSC, CNKI, WEB of SCIENCE: 糖胺聚糖, 肝素, 硫酸化多糖, 氧化, 高碘酸盐, 亚氯酸盐, 半乳糖醛酸, 葡萄糖胺, 离子交换, 药物组合物, 肿瘤, 癌症, 抑制, 实体瘤, 血液肿瘤, 软组织肿瘤, glycosaminoglycans, heparin, sulfated polysaccharides, oxidation, periodate, hypochlorite, galacturonic acid, glucosamine , ion exchange, pharmaceutical, tumor, cancer, inhibition, solid tumor, hematological tumor, soft tissue tumors

**C. DOCUMENTS CONSIDERED TO BE RELEVANT**

| Category* | Citation of document, with indication, where appropriate, of the relevant passages | Relevant to claim No. |
|---|---|---|
| X | US 2021032376 A1 (SHENZHEN HEPALINK PHARMACEUTICAL GROUP CO., LTD.) 04 February 2021 (2021-02-04)<br>description, paragraphs 0029-0059, 0077-0083, and 0089-0095, table 1, and embodiment 1 | 1-10 |
| X | WO 2021128253 A1 (SHENZHEN HEPALINK PHARMACEUTICAL GROUP CO., LTD.) 01 July 2021 (2021-07-01)<br>description, page 2, last paragraph-page 8, paragraph 10, and embodiment 1 | 1-10 |
| X | CN 105744940 A (CT ALTA TECNOLOGIA ISTITUTO DI RICERCHE CHIMICHE E BIOCHIMICHE G RONZONI S R) 06 July 2016 (2016-07-06)<br>description, paragraphs 0028-0049 | 1-10 |

☐ Further documents are listed in the continuation of Box C.

☑ See patent family annex.

* Special categories of cited documents:

- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 February 2025** | **19 February 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/130179**

| Patent document cited in search report | Publication date (day/month/year) | Patent family member(s) | Publication date (day/month/year) |
|---|---|---|---|
| US 2021032376 A1 | 04 February 2021 | TW 201934584 A | 01 September 2019 |
| | | CN 111670038 A | 15 September 2020 |
| | | US 11225531 B2 | 18 January 2022 |
| | | TW 798351 B1 | 11 April 2023 |
| | | CN 111670038 B | 26 January 2024 |
| WO 2021128253 A1 | 01 July 2021 | CN 114901702 A | 12 August 2022 |
| | | CN 114901702 B | 09 January 2024 |
| CN 105744940 A | 06 July 2016 | TR 201906180 T4 | 21 May 2019 |
| | | TR 201802314 T4 | 21 March 2018 |
| | | DK 3520797 T3 | 09 November 2020 |
| | | PL 3520797 T3 | 08 February 2021 |
| | | PL 3065747 T3 | 30 May 2018 |
| | | DK 3065747 T3 | 12 February 2018 |
| | | EP 3520797 A1 | 07 August 2019 |
| | | EP 3520797 B1 | 21 October 2020 |
| | | ES 2835173 T3 | 22 June 2021 |
| | | US 2020172636 A1 | 04 June 2020 |
| | | US 11332548 B2 | 17 May 2022 |
| | | ES 2724475 T3 | 11 September 2019 |
| | | PT 3065747 T | 28 February 2018 |
| | | EP 3308791 A1 | 18 April 2018 |
| | | EP 3308791 B1 | 27 March 2019 |
| | | HK 1222797 A1 | 14 July 2017 |
| | | US 2020190224 A1 | 18 June 2020 |
| | | US 11370848 B2 | 28 June 2022 |
| | | PT 3520797 T | 24 November 2020 |
| | | DK 3308791 T3 | 29 April 2019 |
| | | KR 20160106559 A | 12 September 2016 |
| | | KR 102113905 B1 | 22 May 2020 |
| | | PL 3308791 T3 | 31 July 2019 |
| | | CY 1120221 T1 | 12 December 2018 |
| | | JP 2017508836 A | 30 March 2017 |
| | | JP 6784454 B2 | 11 November 2020 |
| | | ES 2660777 T3 | 26 March 2018 |
| | | CY 1121569 T1 | 29 May 2020 |
| | | EP 3065747 A1 | 14 September 2016 |
| | | EP 3065747 B1 | 06 December 2017 |
| | | US 2016297896 A1 | 13 October 2016 |
| | | US 2017233501 A9 | 17 August 2017 |
| | | US 10822430 B2 | 03 November 2020 |
| | | ITLO 20130006 A1 | 07 May 2015 |
| | | PT 3308791 T | 28 May 2019 |
| | | WO 2015067471 A1 | 14 May 2015 |
| | | CN 105744940 A | 06 July 2016 |
| | | CN 105744940 B | 25 June 2019 |
| | | IN 201647015614 A | 31 August 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202311472694 A
- CN 105744940 A
- WO 2019149179 A1


### Non-patent literature cited in the description


- Structure and active domains of heparin. **CASU B.** Chemistry and biology of heparin and heparan sulfate. Elsevier, 2005, 1-28
- **CASU B.** ; **LINDAHL U.** Structure and biological interactions of heparin and heparan sulfate.. *Adv Carbohydr Chem Biochem*, 2001, vol. 57, 159-206
- **CASU B.** ; **GENNARO U.** *Carbohydr Res*, 1975, vol. 39, 168-176
- **GUERRINI, M.** ; **GUGLIERI, S** ; **NAGGI, A** ; **SASISEKHARAN, R.** ; **TORRI, G.** Low molecular weight heparins: Structural differentiation by bidimentional nuclear magnetic resonance spectroscopy.. *Seminars in Thrombosis and Hemostasis*, 2007, vol. 33, 478-487
- *Remington's Pharmaceutical Sciences*, 1990
- **MAURI, L. et al.** Qualification of HSQC methods for quantitative composition of heparin and low molecular weight heparins. *Journal of Pharmaceutical and Biomedical Analysis*, 2017, vol. 136, 92-105